(19) **Europäisches Patentamt**
**European Patent Office**
**Office européen des brevets**

(11) **EP 1 551 290 B1**

(12) **EUROPÄISCHE PATENTSCHRIFT**

(45) Veröffentlichungstag und Bekanntmachung des Hinweises auf die Patenterteilung:
**27.08.2008 Patentblatt 2008/35**

(51) Int Cl.:
**A61B 5/053** (2006.01)

(21) Anmeldenummer: **03747693.4**

(22) Anmeldetag: **07.10.2003**

(86) Internationale Anmeldenummer:
**PCT/AT2003/000302**

(87) Internationale Veröffentlichungsnummer:
**WO 2004/030535 (15.04.2004 Gazette 2004/16)**

(54) **IMPEDANZBASIERTES MESSVERFAHREN FÜR HÄMODYNAMISCHE PARAMETER**

IMPEDANCE-BASED MEASURING METHOD FOR HEMODYNAMIC PARAMETERS

PROCEDE DE MESURE DE PARAMETRES HEMODYNAMIQUES FONDE SUR L'IMPEDANCE

(84) Benannte Vertragsstaaten:
**AT BE BG CH CY CZ DE DK EE ES FI FR GB GR HU IE IT LI LU MC NL PT RO SE SI SK TR**

(30) Priorität: **07.10.2002 AT 15172002**

(43) Veröffentlichungstag der Anmeldung:
**13.07.2005 Patentblatt 2005/28**

(73) Patentinhaber: **CNSystems Medizintechnik GmbH 8020 Graz (AT)**

(72) Erfinder:
• **SKRABAL, Falko**
**A-8043 Graz (AT)**

• **FORTIN, Jürgen**
**A-8020 Graz (AT)**

(74) Vertreter: **Ellmeyer, Wolfgang Patentanwalt, Mariahilferstrasse 50 1070 Wien (AT)**

(56) Entgegenhaltungen:
**US-A- 4 450 527    US-A- 4 947 862**
**US-A- 4 951 682    US-A- 5 063 937**
**US-A- 5 109 870**

EP 1 551 290 B1

**Beschreibung**

Die Erfindung betrifft ein Verfahren zur Messung des Volumens, der Zusammensetzung und Bewegung von elektrisch leitenden Körperflüssigkeiten, beruhend auf der elektrischen Impedanz des Körpers oder eines Körpersegments, insbesondere für die Elektromechanocardiographie (ELMEC)- bzw. Impedanz-Kardiographie (IKG)-Messung zur Bestimmung haemodynamischer Parameter, wobei durch an der Körperoberfläche angelegte Mess-Elektroden ein Wechsel-Messstrom zumindest einer Frequenz in den Körper eingebracht wird.

In der Medizin besteht häufig die Notwendigkeit der Messung der mechanischen Aktion des Herzens. So wird mittels verschiedener Verfahren, wie z.B. Echokardiographie, mit oder ohne Farbdoppler die Schlagkraft, die Inotropie, die Kontraktilität und die Auswurffraktion (Ejection Fraction) gemessen. Weiters wird häufig die Menge des Blutes, das innerhalb eines Herzschlages vom Herz ausgeworfen wird, das Schlagvolumen und andere haemodynamische Parameter, ermittelt. Daraus lässt sich dann bei Kenntnis der Herzfrequenz das Herzzeitvolumen (HZV = Herzminutenvolumen = Cardiac Output = CO) errechnen. Aus den erwähnten Größen lässt sich die Funktion des Herzens ableiten und es lässt sich die Diagnose von Herzkrankheiten stellen bzw. können neue physiologische Erkenntnisse gewonnen werden. Jedoch kann die Überwachung von schwer herzkranken Patienten auf Intensivstationen oder während der Narkose mittels der Echokardiographie nicht wirklich erfolgen, weil ständig ein Untersuchender anwesend sein müsste. Wegen der Wichtigkeit dieses Problems gibt es auch zahlreiche weitere Methoden zur Messung des HZV in der Medizin. So wird z.B. ein Katheter in die Arteria pulmonalis und/oder in die Aorta eingeführt und dort wird mittels einer Indikatorgröße oder -substanz, die z.B. Wärme, Kälte, Kochsalz oder Lithium sein kann, innerhalb einer Messstrecke der Abfall der Konzentration erwähnter Indikatorsubstanz gemessen und anschließend mittels des Fick'schen Prinzips das HZV gemessen. Der Nachteil dieser Methode ist die Einführung eines Katheters in ein menschliches Gefäß mit allen daraus resultierenden Komplikationen, wie Blutung und Infektion. Es handelt sich damit um ein invasives Verfahren, das hohe Kosten wegen der Einmalkatheter und hohe Gefahren für den Patienten mit sich bringt [i, ii]. Außerdem hat auch das Prinzip der Thermodilution bzw. der Farbstoffverdünnung eine große Fehlerbreite, so dass meist der Mittelwert von mehreren Messungen verwendet wird, um einen plausiblen Wert zu erhalten. Unter körperlicher Belastung, bzw. bei anderen Zuständen, bei denen sich die Körpertemperatur ändert, liefert auch die Thermodilution falsche Werte.

Im jüngster Zeit wurde versucht, auch das Fick'sche Prinzip für die Messung des Herzzeitvolumens durch Messung von Gasen in der Atemluft anzuwenden. Dies ist deswegen möglich, weil ein sehr rascher Gasaustausch zwischen Blut und Atemluft erfolgt, so dass man die Konzentrationen in diesen beiden Medien faktisch gleichsetzen kann. Wenn nun der Atemluft ein Gas beigemischt wird, erhöht sich dessen Konzentration auch im Blut, beendet man die Beisetzung des Gases, dann kommt es zu einem Abfall des Gases im Blut und auch in der Atemluft, wobei aus dem Abfall der Konzentration in der Zeiteinheit wiederum nach dem Fick'schen Prinzip das HZV gemessen werden kann. Eine Methode die sich dabei speziell bewährt hat, ist das $CO_2$ Rebreathing. Dabei wird eine Schleife (Loop) in die Atemwege des Patienten eingebracht und der Patient atmet eine bestimmte Zeit seine eigene ausgeatmete Luft wieder, so dass es zu einem Anstieg der $CO_2$-Konzentration im Blut kommt. Der Nachteil dieser Methoden ist, dass der Patient mit einem Mundstück versehen werden muss und eine möglichst konstante Atmung erfolgen muss, damit eine gleichmäßige Konzentration der Atemgase in der Atemluft und im Blut gewährleistet wird. Darum wird dieses Verfahren hauptsächlich in der Narkose mit konstantem Atemzugvolumen und konstanter Atemfrequenz angewendet. Bei spontan atmenden Patienten bleibt der Nachteil der Atmung durch ein Röhrensystem mit Mundstück erhalten, das den Totraum der Atmung beträchtlich erhöht und auch den Atemwiderstand und damit die Anstrengung der Atmung vergrößert. Außerdem nimmt die Genauigkeit der Methode bei Spontanatmung stark ab. Ein weiteres Verfahren besteht aus einer ähnlichen Methode, wobei statt des $CO_2$ ein inertes Gas, das inhaliert wird und das ebenfalls mit dem Blut rasch equilibriert, für die Messung des HZV verwendet wird.

Ein weiteres Verfahren ist die Messung des Schlagvolumen und anderer haemodynamischer Parameter aus der Pulsform, die an einer peripheren Arterie abgenommen wird. Eine Änderung der Pulsform ist auch durch eine Änderung des Schlagvolumen und anderer haemodynamischer Parameters bedingt, woraus sich indirekt die Änderung des Schlagvolumen und anderer haemodynamischer Parameters über eine Transferfunktion ableiten läßt. Diese Methode muss am Anfang einmal mit einem der oben beschriebenen Verfahren kalibriert werden, sie ist außerdem nicht hinreichend genau. Eine weitere bekannte Methode ist das Messen einer Indikatorsubstanz wie z.B. Indigogrün transkutan an den Kapillaren des Ohres oder Finger, was die Genauigkeit des Fick'schen Prinzips sehr verringert.

Eine weitere aus dem Stand der Technik bekannte Methode ist die Impedanzkardiographie IKG. Bei dieser wird ein konstantes Wechselstromfeld an den Thorax angelegt, und die Änderung der Wechselspannung, die durch dieses Wechselstromfeld entsteht, weist auf eine Änderung des Flüssigkeitsgehaltes im Thorax hin. Genauer gesagt wird der Wechselstromwiderstand (Impedanz) mit dieser Methode gemessen, der ein Maß für die Änderung des thorakalen Flüssigkeitsgehaltes darstellt. Diese Änderung des Flüssigkeitsgehaltes im Thorax wiederum dient als Maß für die pro Schlag ausgeworfene Menge an Blut. Aus dem Schlagvolumen und anderer haemodynamischer Parameter (SV) und der Herzfrequenz (HR) lässt sich dann das Herzzeitvolumen (HZV = SV*HR) errechnen.

Üblicherweise wird dazu ein Elektrodenpaar, das den Strom zum Körper führt, oberhalb oder an der oberen und unterhalb oder an der unteren Thoraxbegrenzung angebracht. Innerhalb dieses Elektrodenpaares wird ein zweites Elektrodenpaar zur Messung der resultierenden Wechselspannung angebracht. Bei diesem inneren Elektrodenpaar muss der richtige gegenseitige Abstand eingehalten werden und zwar muss sich die obere Spannungselektrode zumindest in der Höhe der Glottis befinden und die untere Elektrode in der Höhe des Xiphoids. Der Abstand zwischen beiden Elektroden ist also abhängig von der Länge des Thorax und wird als Elektroden-Messlänge L in der weiteren Folge beschrieben. Man berechnet die Impedanz $Z(t) = u(t)/I_0$, wobei $u(t)$ die sich ändernde Wechselspannung und $I_0$ der in den Körper eingeprägte Wechselstrom mit konstanter Effektivstromstärke ist.

Bisher wurden für diesen Zweck entweder Zirkulärelektroden oder auch Punktelektroden, ähnlich wie EKG-Elektroden verwendet. In der Patentanmeldung "Medizinische Elektrode" [iii] wurde eine neue Elektrodenanordnung beschrieben, bei der auf der selben Folie über eine kurze Strecke, jeweils parallel zwei Bandelektroden angebracht sind, deren Abstand durch die gemeinsame Trägerfolie genau vorgegeben und reproduzierbar ist. Eine dieser parallel verlaufenden Bandelektroden, die auf dieser gemeinsamen Trägerfolie aufgebracht ist, dient der Aufbringung des Messstromes, die andere parallel verlaufende Bandelektrode ist für die Ableitung der Messspannung vorgesehen. Das obere Elektrodenpaar (oder Doppelelektrode) kann z.B. am Nacken, die unteren Elektrodenpaare jeweils links und rechts an der unteren Thoraxapertur angebracht werden. Diese Elektrodenanordnung zeigt eine wesentlich bessere Reproduzierbarkeit der Ergebnisse als die früher verwendeten Ringelektroden bzw. auch besser als die in der Patentanmeldung US 4.450,527 $S_{RAMEK}$ [iv](Fig. 1a + Fig. 1b) beschriebenen Spotelektroden.

Nachteile der beschriebenen Impedanzverfahren waren bisher, dass die Ergebnisse entweder nach der KUBICEK-Gleichung [v, vi] bzw. nach der SRAMEK-Gleichung [4, vii, viii] errechnet wurden, die beide mit sehr vereinfachenden Annahmen über den menschlichen Körper hergeleitet wurden. Diese Annahmen sind nur bedingt richtig, deshalb ergibt sich auch ein beträchtlicher Fehler in der Berechnung des Schlagvolumen und anderer haemodynamischer Parameters und des Herzzeitvolumens.

Gleichung 1 zeigt die KUBICEK-Gleichung zur Berechnung des Schlagvolumen und anderer haemodynamischer Parameters aus der Änderung des Impedanzsignals:

$$SV = \rho * \frac{L^2}{Z_0^2} \cdot LVET \cdot (dZ/dt)_{max} \qquad (1)$$

Wobei mit L die in [cm] an der Körperöberfläche gemessene Messlänge zwischen zwei Elektroden, mit p der spezifische Widerstand des Blutes in [$\Omega$cm], mit $Z_0$ die Grundimpedanz in [$\Omega$], mit $(dZ/dt)_{max}$ die maximale Höhe der 1. Ableitung der elektrischen Widerstands- bzw. Impedanzänderung nach der Zeit in [$\Omega$/sec] durch die Herzaktion und mit LVET, die linksventrikuläre Austreibungszeit [sec] bezeichnet wird.

Wie ersichtlich, geht in diese Gleichung einerseits die Elektroden-Messlänge L quadratisch ein, wobei diese Elektroden-Messlänge derzeit an der Thoraxoberfläche gemessen wird. Weiters ist auch der spezifische Widerstand p des Blutes linear in die Formel eingebracht, wobei der spezifische Widerstand des Blutes in erster Linie vom Gehalt des Blutes an roten Blutkörperchen abhängt. Nach dem Gesetz von LAMBERTS ET. AL. [ix] wird p aus dem Hämatokrit Hkt mittels der Formel näherungsweise berechnet

$$\rho = 71{,}24.e^{\,0{,}000358\ Hkt\ hoch\ 2} \qquad (2)$$

oder aus einer ähnlichen Formel geschätzt bzw. auch in anderen Verfahren einfach konstant gehalten. Dabei bleibt unberücksichtigt, dass es nicht nur der Hämatokrit ist, der die Leitfähigkeit des Blutes verändert, sondern auch die Ionenkonzentration im Plasma sowie die darin befindlichen Eiweißstoffe. Deswegen wird aus einer empirisch gewonnenen Gleichung, die nur einen und nicht alle Blutbestandteile berücksichtigt, nie die genaue Leitfähigkeit oder der Widerstand des Blutes zu ermitteln sein. Weiters ändert sich auch die Leitfähigkeit des Blutes durch die Flußgeschwindigkeit, da bei größeren Geschwindigkeiten die Erythrocyten sich im Blutstrom längs ausrichten und damit den Flüssigkeitsquerschnitt vergrößern. Bei noch höheren Geschwindigkeiten und daraus entstehenden Turbulenzen kann der Widerstand des Blutes wieder ansteigen.

In der Formel nach SRAMEK wird statt der Elektroden-Messlänge 17 % der Körpergröße eingegeben, weil sich empirisch herausgestellt hat, dass die Thoraxlänge ca. 17 % der gesamten Körperlänge ausmacht. Eine weitere Annahme bei dieser Formel ist der Divisor 4.25, der sich aus dem geschätzten Zusammenhang zwischen Elektroden-Messlänge und Bauchumfang, sowie aus einem geschätzten konstanten Verhältnis zwischen einem zylindrischen Thoraxmodel und einem kegelstumpfförmigen Thoraxmodel (US 4.450,527, Spalte 5, Zeile 50 ff.) ergibt. Somit geht die Länge des Körpers H sogar kubisch in die Formel ein.

$$SV = \frac{(0.17 \cdot H)^3}{4.25} \cdot LVET \cdot \frac{(dZ/dt)_{max}}{Z_0} \quad (3)$$

Bernstein[*] hat diese Formel noch weiter "korrigiert", indem er die obige Formel noch mit einem Korrekturfaktor δ multipliziert hat.

$$\delta = \beta (W_{real}/W_{ideal}) \qquad (4)$$

wobei es sich bei β um einen "Blood volume index" handelt, und $W_{ideal}$ und $W_{real}$ um das Idealgewicht und Realgewicht eines Menschen handelt.

Das Idealgewicht bei Männern beträgt:

$$W_{ideal} = 0{,}534\,H - 17{,}36 \qquad (5)$$

Das Idealgewicht bei Frauen beträgt:

$$W_{ideal} = 0{,}534\,H - 27{,}36 \qquad (6)$$

H bezeichnet dabei die Größe des Menschen in [cm].

Es wird ersichtlich, dass in allen Gleichungen unterschiedliche Maßeinheiten miteinander vermengt werden. Damit haben die gewonnenen Gleichungen mit echt abgeleiteter, glaubhafter Mathematik nichts zu tun. Durch dieses Einbringen von anthropometrischen Größen in die Gleichung wird auch ein indirektes Maß für das Schlagvolumen und anderer haemodynamischer Parameter des Herzens beim Gesunden direkt in die Berechnung des Schlagvolumen und anderer haemodynamischer Parameters einbezogen. Das HZV passt nämlich beim Gesunden zur Körperoberfläche wie ein Maßanzug. Somit geht ein Parameter in die Formel ein, der mit der *Messung* des Schlagvolumen und anderer haemodynamischer Parameters nichts zu tun hat, nämlich die Körpermaße des Patienten. Deshalb hat ein großer Patient automatisch durch das Einbringen der Körpergröße bzw. der von der Körpergröße abhängigen Elektroden-Messlänge L zwischen Glottis und Xiphoid in die Berechnungsformel ein größeres Schlagvolumen und andere, größere haemodynamische Parameter als ein kleiner Patient. Auch in der Formel von Kubicek die oben angeführt wurde, geht ein Maß für die Dimensionen des Körpers direkt in die Formel ein.

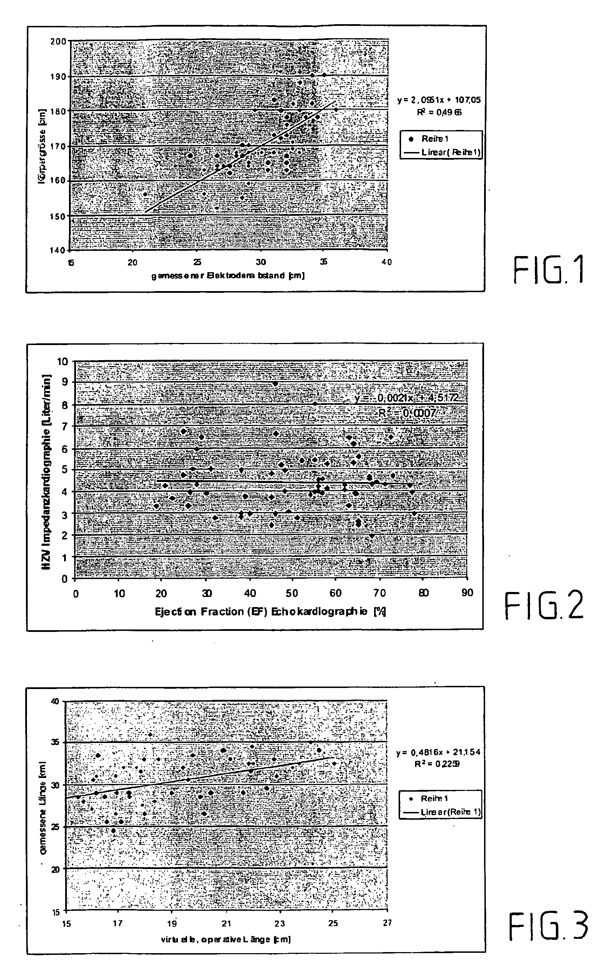

Wie in Fig.1 gezeigt ist, korreliert nämlich die Messlänge zwischen den Elektroden bei richtiger Aufbringung an der oberen und unteren Thoraxapertur überraschend gut mit der Grösse des Patienten.

Bei Herzgesunden ist die Übereinstimmung von blutig gemessenem Schlagvolumen und anderer haemodynamischer Parameter mit der Impedanzkardiographie nach obigen Ausführungen deswegen gut, weil tatsächlich die Körpergröße ein Maß für das Herzzeitvolumen ist. Ein großer schwerer Mensch muss tatsächlich in der Zeiteinheit viel mehr Blut zu den Geweben führen, als ein kleiner, zart gebauter Mensch. Bei Herzkranken ist dieses Prinzip nicht mehr aufrecht, deswegen ist die Korrelation zwischen dem tatsächlichen Herzzeitvolumen und den mittels der Impedanzkardiographie gemessenem Herzzeitvolumen sehr schlecht oder nicht existent, weil plötzlich den Körpermassen nicht mehr die in die Formel eingebrachte Bedeutung zukommt. Es entsteht daher bei Herzkranken ein enormes Bias in Richtung normaler und damit fälschlich hoher Werte.

Zusätzlich wird der mittels Impedanzkardiographie ermittelte Wert durch folgendes Phänomen zusätzlich in die falsche Richtung, nämlich in jene zu hoher HZV-Werte verfälscht. Bei Patienten mit Herzinsuffizienz befindet sich aufgrund der Krankheit in den meisten Fällen mehr Körperwasser im Thorax, als bei Herzgesunden. Dieser erhöhte thorakale Flüssigkeitsgehalt senkt naturgemäß die Grundimpedanz $Z_0$ in Ω. Dieser Wert geht invertiert (SRAMEK) bzw. quadratisch invertiert (KUBICEK) in die jeweiligen Berechnungsformeln ein und verfälscht den berechneten Wert des HZV nach oben, was zu fatalen Fehldiagnosen führen kann. Bei Herzgesunden ist $Z_0$ ein Maß für die Thoraxgeometrie, bei herzinsuffizienten Patienten mit erhöhtem thorakalen Flüssigkeitsgehalt ist dies eben nicht der Fall.

Dies wird in Fig. 2 anschaulich demonstriert. Hier wurde an Patienten mit und ohne Herzinsuffuzienz die Auswurffraktion (Ejection Fraction EF) mittels der Simpsonmethode echokardiographisch gemessen und die Auswurffraktion mit dem HZV verglichen. Der echokardiographische Parameter EF wurde deswegen statt des echokardiographisch gemessenen HZV gewählt, weil er wesentlich präziser zu messen ist. Wie ersichtlich besteht keinerlei Zusammenhang zwischen HZV und Auswurffraktion, was man an und für sich erwarten würde, wenn die Impedanzkardiographie bei Herzinsuffizienz Aussagekraft hätte.

Deswegen hat sich die Impedanzkardiographie bei den Kardiologen zumindest in Europa nicht wirklich durchgesetzt, weil die Übereinstimmung mit dem tatsächlichen Schlagvolumen und anderen haemodynamischen Parametern zwar bei Gesunden gut sein mag, bei Herzkranken, wo der Wert wirklich für die Diagnose interessiert, ist jedoch die Genauigkeit sehr schlecht. In Amerika wird die Methode trotzdem jetzt deswegen vermehrt eingesetzt, weil es sich gezeigt hat, dass die Relativänderungen des Schlagvolumen und anderer haemodynamischer Parameter sich gut verfolgen lassen, sodass man die Auswirkung pharmakologischer Interventionen gut verfolgen kann auch wenn die Absolutwerte falsch sein mögen.

Keines der am Markt befindlichen Geräte würde außerdem einen Wert des Schlagvolumen und anderer haemodynamischer Parameters oder des Herzzeitvolumen ausgeben können, wenn nicht vorher zumindest die Körpergröße, oder die Thoraxlänge zwischen den Elektroden, also ein anderes Maß für die Körpergröße eingegeben wird. Gerade auf Intensivstationen besteht oft auch keine Möglichkeit Gewicht und Größe zu messen oder erfragen. Sollte ein falscher Wert eingeben werden, was in der Praxis leicht passieren kann, würde das Ergebnis noch zusätzlich weiter verfälscht.

Es sollte jedoch eine Vorrichtung bzw. ein Verfahren zur Bestimmung des Herzzeitvolumen auch einen verläßlichen Wert liefern, wenn kein *a priori* Wissen über Körpergröße und Gewicht vorhanden ist, wie dies selbstverständlich auch beim Goldstandard der Thermodilution, bzw. auch bei anderen Methoden, die das Fick'sche Prinzip verwenden, wie die Methode des $CO_2$ Rebreathing oder andere Atemgasmethoden, möglich ist. Sobald *a priori* Wissen über die Körpermaße mitverwendet wird, wird das Messergebnis auch schon in die Richtung, in die der Messwert des Cardiac Output gehen sollte, beeinflusst, das heißt, dass *Bias* in die Gleichung eingebracht wird, welches beim herzgesunden Menschen fälschlich gute Ergebnisse der Methode vortäuscht. Außerdem sollten bei einem elektrisch *gemessenen Herzzeitvolumen* auch nur *elektrisch gemessene Parameter* in der Gleichung Platz finden.

Bei der in der US 4 450 527 A beschriebenen Vorrichtung zur Impedanzmessung müssen die Dimensionen des Brustkorbes, insbesondere die Messlänge zwischen den Elektroden, an denen gemessen wird, ermittelt und eingegeben werden. Es wird die Thoraximpedanz in Abhängigkeit von der Zeit gemessen und die durch die Atmungsbewegung hervorgerufenen Effekte eliminiert, sodass während des Messvorganges eine normale Atmung des Patienten ermöglicht wird. Punktförmige Elektroden zur Stromeinprägung einerseits und zum Abgriff einer Messspannung werden im Halsbereich und im Brustbeinbereich angebracht. Die Messlänge zwischen den unteren und den oberen Elektroden wird während der Messung nicht verändert.

In der US 5 109 870 A ist ein Katheter zur Messung der Motilität und Peristaltik in rohrenförmigen Organen, z.B. Speiseröhre, durch gleichzeitige, vielfache Impedanzmessungen gezeigt, der eine Isolations-Kunststoffröhre, ringförmige Elektroden und Innenkanäle für die Elektrodenanschlüsse aufweist. Die ringförmigen Elektroden sind an Impedanz-Transformatoren angeschlossen, welche die gemessenen Signale in Spannungs- und Stromsignale wandeln, um sie so anzeigen zu können. Aufgrund dieser Mehrfach-Elektrodenanordnung ist eine Simultanmessung einer Vielzahl von Messkanälen durchführbar, um Rückschlüsse auf die Bewegungs- und Transportcharakteristik des Organs zu erhalten. Zu diesem Zweck muß der Katheter in das Organ eingeführt und in diesem in einer bestimmten Position festgelegt werden. Selbst für einen Patienten, der sich in einem stabilen gesundheitlichen Zustand befindet, ist dieses Messverfahren sehr anstrengend und kann daher nicht beliebig oft wiederholt werden. Das Herzzeitvolumen wird mit der angegebenen Methode nicht bestimmt.

Weiters ist in der US 4 951 682 A ein Herzkatheter zur Messung des Herzzeitvolumens mit mehreren voneinander beabstandeten Ringelektroden angegeben. Dabei wird in der Einleitung (Spalte 2, zweiter Absatz) dieses Dokuments auf nicht-invasive Methoden zur Ermittlung des Herzzeitvolumens eingegangen und festgestellt, daß diese alle gravierende Nachteile hätten und die invasive Messung mittels Herzkatheter demgegenüber entscheidende Vorteile hätte. Es wird allein auf eine invasive Herzkathetermessung verwiesen, die naturgemäß nicht sehr oft bei einem Patienten ausgeführt werden und erhebliche Komplikationen mit sich bringen kann.

Aus der US 4 947 862 A geht eine Vorrichtung zur Bestimmung des Körperfettanteils eines Patienten hervor, bei der ein hochfrequenter Strom in den Körper eingeleitet und eine Spannung abgegriffen wird. Über Amplituden- und Phasen-Detektionskreise werden Amplitude und Phase der erzeugten Spannungssignale gegenüber dem eingeprägten Strom gemessen. Körpergröße, Gewicht und Alter müssen dabei bestimmt und in eine Eingabevorrichtung eingegeben werden, womit Messfehler dieser Werte in die Berechnung des Körperfettanteils eingehen.

Schließlich ist in der US 5 063 937 A noch ein Mehrfach-Frequenz-Messsystem zur Ermittlung der Bio-Impedanz eines Patientenkörpers über einen großen Frequenzbereich angegeben, wobei Fehler bei der Bestimmung der Messlänge zwischen den Elektroden keine Berücksichtigung finden.

Aufgabe der Erfindung ist es daher, ein Verfahren der eingangs genannten Art, insbesondere zur Bestimmung des Schlagvolumens bzw. ganz allgemein auch zur Messung von anderen haemodynamischen Parametern wie Inotropie, Auswurffraktion, mittels der Impedanzkardiographie anzugeben, das die vorgenannten Nachteile vermeiden hilft.

Erfindungsgemäß wird dies die Merkmale des Anspruchs 1 erreicht.

Durch die Messung der Impedanz bzw. deren Änderung bei zwei unterschiedlichen Messlängen läßt sich die tatsächlich elektrisch partizipierende "operative Körpersegmentlänge" bzw. das tatsächlich partizipierende elektrische "operative Körpersegmentvolumen" bzw. der tatsächlich gemessene "operative spezifische Widerstand" des Blutes mit Lösungen von Gleichungen mit mehreren Unbekannten elektrisch ermitteln oder aber mit empirisch gewonnenen Gleichungen diese zusätzliche Information in einem sogenannten "black box"-Modell einbringen, bei dem nur mehr elektrisch gemessene Größen eingehen. Die Abhängigkeit von Körperabmessungen und anderen Körpermessgrößen ist dadurch beseitigt.

Da insbesondere bei der Bestimmung des Herzzeitvolumens die Impedanzänderungen im Brustbereich eine wesentliche Aussagekraft haben und es sich als günstig erwiesen hat, die Veränderung der Elektroden-Messlänge in Hauptflussrichtung des Blutes vorzunehmen, kann in Weiterbildung der Erfindung vorgesehen sein, dass die Impedanz am Brustkorb nahe der oberen und der unteren Thoraxapertur bei zumindest zwei verschiedenen Messlängen des im wesentlichen selben Körpersegments abgegriffen wird.

Eine andere Variante der Erfindung, bei der die Impedanz am Rumpf nahe des oberen und unteren Ende des Rumpfes bei zumindest zwei verschiedenen Messlängen des im wesentlichen selben Körpersegments abgegriffen wird, hat den Vorteil, dass auch die Impedanz der Extremitäten bei zwei unterschiedlichen Distanzen vermessen werden kann, sodass auch für diesen Bereich eine operative Länge bzw. ein elektrisch partizipierendes Volumen ermittelt werden kann.

Bei Einleitung des Messstromes im Thorax- oder Rumpfbereich ergeben sich im Körperinneren Strombäuche, während für die Messung eine möglichst geradlinige Stromausbreitung vorteilhaft ist, wie sie durch Stromeinbringung über die Körperenden erreicht werden kann. Eine weitere Variante der Erfindung kann daher darin bestehen, dass der Strom statt an der unteren Thoraxapertur am oder nahe am unteren Körperende eingebracht und die Impedanz am Thorax und/oder am Rumpf und/oder an den Extremitäten, jeweils bei zumindest zwei verschiedenen Messlängen gemessen wird.

Für die Bestimmung einiger haemodynamischer Parameter bzw. Parameter für den Flüssigkeitshaushalt kann es weiters vorteilhaft sein, wenn zusätzlich die Ganzkörperimpedanz, also die Impedanz zwischen unterem und oberem Körperende gemessen wird. Diese zusätzliche Messung der Ganzkörperimpedanz kann nicht nur auf das erfindungsgemäße Verfahren sondern auch auf jedes andere konventionelle Verfahren der Impedanzkardiographiemessung angewandt werden, es wird für diese unabhängig von der erfindungsgemäßen Meßmethode mit unterschiedlicher Elektroden-Messlänge Schutz beansprucht, also auch für die bereits aus dem Stand der Technik bekannten gattungsbildenden Verfahren.

Größtmögliche Genauigkeit und Reproduzierbarkeit der Impedanzmessung ist ferner dann gegeben, wenn die Längendifferenz zwischen den beiden verschiedenen Messlängen klein im Verhältnis zur Länge des gemessenen Körperteils ist, insbesondere wenn das Verhältnis der Länge des untersuchten Körperteils zur Längendifferenz zwischen 3:1 und 50:1, in besonders bevorzugter Weise ungefähr 10:1, beträgt.

Bei der Impedanzmessung am Körper kann über Strom-Elektroden, die auf der Körperoberfläche durch eine Stromelektroden-Messlänge voneinander beabstandet sind, ein Wechsel-Messstrom eingeprägt und über Spannungs-Elektroden, die auf der Körperoberfläche, insbesondere auf der Thoraxoberfläche, durch eine Spannungselektroden-Messlänge voneinander beabstandet sind, eine durch den Messstrom hervorgerufene Messspannung abgegriffen werden, und aus dem Messstrom und der Messspannung die elektrische Impedanz bzw. deren zeitliche Änderung berechnet werden.

Eine Ausführungsform der Erfindung kann darin bestehen, dass aus den bei unterschiedlichen Messlängen zwischen Elektroden ermittelten Impedanzwerten eine operative Elektroden-Messlänge bzw. gegebenenfalls zusätzlich ein operativer Elektrodenabstand berechnet wird. Über diese tatsächlich im Körper wirksamen Größen kann eine verläßliche Bestimmung der Impedanz oder deren Änderung erzielt werden. Auf diese Weise kann auf die händische Vermessung der Änderungen in der Elektroden-Messlänge verzichtet werden.

Es kann somit die elektrisch operative Länge des Körpersegments aus der Formel $L_0 = d/(Z_{02}/Z_{01} - 1)$ errechnet werden, wobei mit d die Längendifferenz zwischen den beiden zur Messung herangezogen Elektroden-Messlängen, mit $Z_{02}$ die Impedanz über die längere Elektroden-Messlänge und mit $Z_{01}$ die Impedanz über die kürzere Elektroden-Messlänge bezeichnet ist.

Um eine asymmetrische Stromverteilung im Körper des Patienten zu vermeiden, kann gemäß einer anderen Ausführungsform vorgesehen sein, dass die Spannungselektroden an der unteren Thoraxapertur als Doppelelektroden jeweils links und rechts seitlich am Thorax ausgeführt sind, wobei jeweils die in der Längsrichtung auf selber Längendistanz liegenden Elektroden elektrisch miteinander verbunden sind.

Alternativ kann vorgesehen sein, dass wahlweise die jeweils links bzw. rechts an der Thoraxapertur liegenden Elektroden weggeschaltet werden können.

Bei der Anbringung von Messelektroden am Rumpf kann eine vorteilhafte Ausführungsform des erfindungsgemäßen Verfahrens darin bestehen, dass die Spannungselektroden am unteren Ende des Rumpfes als Doppelelektroden jeweils links und rechts am unteren Ende des Rumpfes ausgeführt sind, wobei jeweils die in der Längsrichtung auf selber Längendistanz liegenden Elektroden elektrisch miteinander verbunden sind. Damit kann ein relativ großes Körpervolumen von der Messung erfasst werden. Dabei können alternativ wahlweise die jeweils links bzw. rechts am unteren Ende des Rumpfes liegenden Elektroden weggeschaltet werden.

Durch die Anbringung zumindest einer zusätzlichen Stromelektrode und/oder Spannungselektrode ergibt sich eine veränderte Elektrodenmesslänge der Stromelektroden und/oder Spannungselektroden in Bezug auf ein weiteres Elektrodenelement, vorzugsweise in der Längsrichtung des Körpers, und damit in die Hauptflussrichtung des Blutes, sodass gleichzeitig oder abwechselnd die Messung der Impedanz und deren Änderung im Thorax jeweils mit der kürzeren und längeren Elektroden-Messlänge erfolgen kann. Diese Veränderung in der Elektroden-Messlänge L sollte konstant, bekannt, oder berechenbar sein.

Es können die Ziele der Erfindung bei einem Verfahren der vorstehend genannten Art aber auch dadurch gelöst werden, dass die elektrische Impedanz bei zwei oder mehreren Messfrequenzen gemessen und die Anteile des Intra- und Extrazellulärraumes bestimmt werden, und dass diese Größen zur Berechnung des Schlagvolumen und anderer haemodynamischer Parameter verwendet werden. Dieses Verfahren kann unabhängig von der erfindungsgemäßen, definierten Veränderung der Messlänge auch in einem gewöhnlichen Zwei-Elektrodensystem oder aber auch in Kombination mit diesem angewandt werden. Durch die Wahl der zwei Messfrequenzen kann die Eigenschaft des Blutes, bei unterschiedlichen Frequenzen einen unterschiedlichen Widerstand anzunehmen, zur Bestimmung verschiedener haemodynamischer Paramter genutzt werden, für welche der spezifische Widerstand des Blutes von Bedeutung ist.

Die Anzahl der unterschiedlichen Frequenzen, die für das erfindungsgemäße Verfahren angewandt werden, ist nach oben hin unbegrenzt, auch ein kontinuierliches Überstreichen eines Frequenzbandes (sweep), vorzugsweise von einer unteren Messfrequenz bis zu einer oberen Messfrequenz, liegt im Rahmen der Erfindung, wobei die untere Messfrequenz gemäß einer bevorzugten Ausführungsform der Erfindung ca. 1 kHz und die obere Messfrequenz maximal ca. 1000 kHz beträgt.

Auch der Phasenwinkel zwischen Messstrom und Messspannung bei unterschiedlichen Frequenzen kann ein Maß für die Bestimmung haemodynamische Parameter sein.

Eine vorteilhafte Ausgestaltung des erfindungsgemäßen Verfahrens kann darin bestehen, dass die elektrische Impedanz bzw. der Impedanzverlauf nach der Zeit bei zwei Messfrequenzen gleichzeitig gemessen wird. Die beiden Frequenzen können z.B. durch Frequenzfilter (Frequenz-Multiplexer) getrennt werden. Alternativ dazu kann in sehr kurzen Zeitfenstern abwechselnd die eine und die andere Messfrequenz gemessen werden. Aus den unterschiedlichen Impedanzwerten, die bei unterschiedlichen Frequenzen gemessen werden können, kann die Körperwasserverteilung bestimmt und auf die Thorax-Geometrie rückgeschlossen werden.

Eine weitere Ausgestaltung des erfindungsgemäßen Verfahrens kann darin bestehen, dass die Impedanz bei drei verschiedenen Frequenzen gemessen wird, wobei die verschiedenen Frequenzen zwischen 1 und 10 kHz, 30 bis 100 kHz und höher als 200 kHz betragen.

Wie bereits erläutert, besteht eines der Probleme der Impedanzkardigraphie in der Bestimmung des spezifischen Blutwiderstandes, der in verschiedenen Berechnungsformeln haemodynamischer Parameter eine Rolle spielt. Da die Abnahme der maximalen zeitlichen Ableitung der Impedanz nach der Zeit mit ansteigenden Frequenzen ein Maß für den spezifischen Widerstand des Blutes ist, können die Ziele der Erfindung bei einem vorstehend genannnten Verfahren dadurch gelöst werden, dass die maximale zeitliche Änderung der gemessenen Impedanzwerte (dZ/dt) bei zumindest zwei unterschiedlichen Messfrequenzen bestimmt und aus diesen der spezifische Widerstand des im Körper befindlichen Blutes ermittelt wird. Eine derartige Messung kann mit oder ohne Veränderung der Messlänge zwischen den Spannungs- oder Strom-Elektroden durchgeführt werden.

Aus den Impedanzänderungen z.B. bei einer hohen und bei einer niedrigen Messfrequenz kann ein Verhältnis berechnet werden, das ein Maß für die durch die Beschleunigung in der Aorta verformten Erythozyten ist. Aus diesem Verhältnis können durch geeignete mathematische Signalanalyse weitere Paramater abgeleitet werden, die für die Berechnung des Herzschlagvolumen bedeutsam sind.

So kann gemäß einer Weiterbildung der Erfindung die maximale zeitliche Änderung des gemessenen Impedanzwertes, insbesondere in relativ schmalen Zeitfenstern, zu unterschiedlichen Zeiten der Herzperiode, bestimmt werden. Auf diese Weise ergibt sich eine zeitliche Mittelung der Impedanzwertänderungen über die Herzperiode.

Eine Mittelwertbildung aus den Extremwerten einer Herzperiode kann gemäß einer weiteren Variante der Erfindung dadurch vorgenommen werden, dass die Zeitfenster bei einem Steilanstieg des spezifischen Widerstandes und zum Zeitpunkt des minimalen Blutflusses am Ende der Diastole festgesetzt werden.

Werden Zeitfenster als schmale Gleitfenster über die gesamte Herzperiode gelegt, so läßt sich die Genauigkeit des erfindungsgemäßen Verfahrens erhöhen.

Schließlich besteht ein bedeutender Vorteil des erfindungsgemäßen Verfahrens darin, dass empirische Gleichungen, die mit Hilfe eines Goldstandards wie z.B. des Fick'schen Prinzipes für das Schlagvolumen, bzw. z.B. der Echocardiographie oder Isotopenmethoden für andere Parameter, wie z.B. Auswurfsfraktion, Pulmonalen Wedge-Druck, diastolische Funktion od.dgl. gewonnen wurden, für die Messung haemodynamischer Parameter bzw. Unterwasserwiegung oder DXA Methoden, bzw. Verdünnungsmethoden für das Körperwasser verwendet werden können. Diese empirischen Gleichungen können z.B. mittels partieller Korrelationen und multipler Regressionsgleichungen oder auch mittels neuronaler Netze bzw. anderer "Machine-Learning"-Methoden gewonnen werden.

Bei Verwendung von zwei beabstandeten Spannungs-Elektroden wird die Messspannung bei einer ersten Spannungselektroden-Messlänge und bei einer von der ersten verschiedenen, zweiten Spannungselektroden-Messlänge bestimmt und aus den Messwerten die für die Impedanzbestimmung operative Länge gegenüber einer Bezugselektrode ermittelt.

Um auch den Abstand zwischen den Spannungselektroden durch Messung elektrischer Größen bestimmen zu können, kann gemäß einer weiteren Ausführungsform der Erfindung die Messspannung weiters bei einer von der ersten und von der zweiten verschiedenen, dritten Spannungselektroden-Messlänge bestimmt werden.

Außerdem kann durch Anbringen von Elektroden an der Peripherie bzw. an den Extremitäten bzw. am oberen und unteren Körperende die Gesamtkörperimpedanz bei verschiedenen Frequenzen, und damit auch das Körperwasser mit seinen Teilfraktionen wie Extrazellulärraum und Intrazellulärraum ermittelt werden und es können aus der Relation von Körperwasser zu dem elektrisch partizipierendem Thoraxvolumen weitere Rückschlüsse auf das tatsächliche Schlagvolumen und andere haemodynamische Parameter gewonnen werden. Wenn zusätzlich auch an den Extremitäten mit verschiedenen Frequenzen gemessen wird, ergibt sich auch die Gelegenheit den Intrazellulärraum und Extrazellulärraum zu ermitteln und auch diese Werte und deren Relation zueinander in eine Gleichung zur Errechnung des HZV einzubringen. Nachdem Intrazellulärraum und Extrazellulärraum bei Herzkrankheiten charakteristisch verändert sind, können weitere wichtige Rückschlüsse über die Funktion des Herzens gewonnen werden. So kommt es bei Herzinsuffizienz zu einer Abnahme des Intrazellulärraums und Zunahme des Extrazellulärraums.

Eine weitere Ausgestaltung der Erfindung kann daher darin bestehen, dass der Messstrom über zwei Strom-Elektroden an den oberen und unteren Enden jeweils zumindest einer Körperextremität, z.B. an einem Bein, z.B. am Knöchel, und/oder an einem Arm, z.B. am Handgelenk, eingeprägt wird.

Alle an der Impedanzmessung im Körper beteiligten Stoffe unterliegen einer Frequenzabhängigkeit, die wertvolle Hinweise auf die Konstitution des zu vermessenden Organismus liefern können.

Es sollte daher der Messstrom bei unterschiedlichen Messfrequenzen eingeprägt und die zugehörigen Messspannungswerte und deren Änderungen über die Zeit, besonders über die Zeit des Herzzyklus bestimmt werden. Die dabei verwendeten Frequenzen sollten eine messbare Veränderung der Impedanzwerte für Blut ergeben.

Ein vorteilhaftes Signal-Rausch-Verhältnis der Messwertbestimmungen läßt sich erzielen, wenn in weiterer Ausbildung der Erfindung der Messstrom bei mehreren unterschiedlichen Spannungselektroden-Messlängen und bei mehreren unterschiedlichen Messfrequenzen eingeprägt und die durch den Messstrom hervorgerufene Messspannung gemessen wird.

Weiters kann es sich für die Bestimmung des Charakters der Impedanz (induktiv oder kapazitiv) als günstig erweisen, den Phasenwinkel zwischen Messstrom und Messspannung zu bestimmen.

Weiters ist es vorteilhaft, wenn Amplituden, Flächen und Anstiegs- bzw. Abfallstangenten der Impedanz-Wellen B, C, X und O einzeln oder gemeinsam zur Berechnung haemodynamischer Parameter verwendet werden.

Eine weitere Variante des erfindungsgemäßen Verfahrens kann darin bestehen, dass das Serumnatrium bestimmt und zur Berechnung der interessierenden Parameter mitverwendet wird.

Es kann auch die Konzentration des Serumnatriums durch das erfindungsgemäße Verfahren mathematisch geschätzt und als Resultat ausgegeben werden.

Weiters können die Hormone, wie z.B. ADH und Natriuretisches Peptid, speziell auch das Atriale Natriuretische Hormon, das "Brain Natriuretic Peptid" und deren Vorstufen, die das Körperwasser, dessen Fraktionen und dessen Zusammensetzung regeln, durch das erfindungsgemäße Verfahren mit Hilfe empirischer Gleichungen geschätzt und als Resultat ausgegeben werden.

Unter Ausnützung moderner Kommunikationsmittel kann die Verarbeitung der aus dem erfindungsgemäßen Verfahren gewonnenen Daten in der Weise geschehen, dass die Resultate des Verfahrens digital an eine zentrale Stelle, vorzugsweise per Telefon oder Email, verschickt werden, wo sie weiter verrechnet und beurteilt werden, und daß dem Patienten die nötigen Maßnahmen und Therapieänderungen von einem entfernten Ort aus mitgeteilt werden.

Weiters betrifft die Erfindung ein Gerät zur Messung der elektrischen Impedanz bzw. deren zeitliche Änderung an einem menschlichen Körper, insbesondere für eine Elektromechanocardiographie- bzw. Impedanz-Kardiographie (IKG)-Messung zur Bestimmung haemodynamischer Parameter.

Erfindungsgemäß wird die bereits eingangs definierte Aufgabenstellung der Erfindung dadurch gelöst, dass zwei Spannungselektroden vorgesehen sind, von denen zumindest eine der beiden als Doppelspannungs-Elektrodenelement ausgeführt ist, wobei die Impedanz und deren Änderung nach der Zeit zwischen den zwei Spannungselektroden abgreifbar ist.

Mit Hilfe der einen Spannungselektrode in Form einer Doppelspannungs-Elektrode, die zwei in bekanntem Abstand zueinander angeordnete Elektroden aufweist, ist die Differenz zweier Elektroden-Meßlängen in Bezug auf die andere Spannungselektrode festgelegt, sodaß aus zwei an der Doppelspannungs-Elektrode abgegriffenen Meßspannungen eine operative Elektroden-Messlänge bestimmt werden kann.

Eine an die Körperform anpassbare Ausführung des Doppelspannungs-Elektrodenelements kann erreicht werden, wenn das zumindest eine Doppelspannungs-Elektrodenelement auf einer gemeinsamen, isolierenden Trägerfolie aufgebracht ist.

In weiterer Ausbildung der Erfindung kann zumindest eine der Spannungselektroden aus einem Dreifachelektrodenelement bestehend aus einer Stromelektrode und zwei Spannungselektroden gebildet sein. Auf diese Weise kann die Einbringung des Wechselmessstromes und das Abgreifen von Messspannungen bei der Durchführung des erfindungsgemäßen Verfahrens an einem einzigen Elektrodenelement erfolgen. Insbesondere kann die Stromelektrode und das Doppelspannungselektrodenelement als Dreifach-Elektrodenelement auf einer gemeinsamen Trägerfolie aufgebracht sein.

Um die Impedanzmessung gemäß dem erfindungsgemäßen Verfahren ohne händisches Umstecken oder Umschalten durchführen zu können, kann weiters vorgesehen sein, dass alle Anschlüsse der Elektroden-Elemente über Anschlußleitungen in einem Verteilerstück zusammengeführt sind, und dass das Verteilerstück mit Messleitungen und Steuerleitungen einer Messvorrichtung verbunden ist.

Eine weitere Möglichkeit der Automatisierung des erfindungsgemäßen Verfahrens kann darin bestehen, dass das Verteilerstück steuerbar ist, sodass die Elektroden-Elemente mit verschiedenen Messleitungen und Steuerleitungen der Messvorrichtung verbindbar sind.

Da die verschiedenen, durch das erfindungsgemäße Messsystem messbaren Impedanzwerte von der Lage des menschlichen Körpers im Raum abhängen, ist es vorteilhaft, den Winkel zwischen der Körperlängsachse und der Waagrechten oder Senkrechten aufzuzeichnen. Eine weitere Ausbildung des erfindungsgemäßen Messsystems kann daher darin bestehen, dass ein Winkelmesser zur Messung der Körperneigung vorgesehen ist. Dieser kann vorzugsweise auf dem Verteilerstück angeordnet sein.

Weiters betrifft die Erfindung ein medizinisches Elektroden-Element zur Messung der elektrischen Impedanz bzw. deren zeitlichen Änderung an einem menschlichen Körper, insbesondere für eine Elektromechanocardiographie oder Impedanz-Kardiographie (IKG)-Messung zur Bestimmung haemodynamischer Parameter, mit einer ersten Strom-Elektrode, die einen Strom-Anschluss zum Einprägen eines elektrischen Wechsel-Messstromes aufweist, und einer von dieser beabstandeten, ersten Spannungs-Elektrode, die einen Spannungs-Anschluss zum Abgreifen einer elektrischen Messspannung aufweist, wobei zumindest eine weitere Spannungs-Elektrode mit einem Spannungs-Anschluss vorgesehen ist, und wobei die zumindest eine weitere Spannungs-Elektrode in einem Abstand zur ersten Spannungs-Elektrode angeordnet ist bzw. sind, zur Durchführung des erfindungsgemäßen Verfahrens, wobei die erste Spannungs-Elektrode sowie die zumindest eine weitere Spannungs-Elektrode in Form von zueinander parallelen, elektrisch leitenden Streifen ausgebildet sind, und die Streifenbreite gleich oder bevorzugt kleiner als der Abstand zwischen den Streifen ist.

Durch die streifenförmige Ausbildung der Elektroden ergibt sich ein relativ hohes Messvolumen, z.B. innerhalb des Thorax, und es lassen sich damit aussagekräftige Messwerte zur Bestimmung der Körperimpedanz erzielen.

Es ist somit im Gegensatz zur konventionellen Vierpunktmethode (zwei Stromelektroden und zwei Spannungselektroden) zumindest eine zusätzliche Spannungs-Elektrode bzw. eventuell auch zusätzliche Strom-Elektrode vorhanden, die so am Körper angebracht wird bzw. werden, dass sich durch die Anbringung dieser zusätzlichen Spannungs-Elektrode und/oder Strom-Elektrode eine Veränderung der Messlänge L zwischen den Spannungselektroden bzw. auch des Einbringungsortes des Stromes zumindest zweier am Körper des Patienten angebrachter Elektroden-Elemente, vorzugsweise in der Längsrichtung des Körpers und damit in die Hauptflussrichtung des Blutes ergibt und dass gleichzeitig oder abwechselnd die Messung der Impedanz und deren Änderung über die Zeit über das untersuchte Körpersegment - jeweils mit der kürzeren und längeren Messlänge zwischen den jeweiligen Elektroden erfolgen kann.

Durch Einführung von zusätzlichen Freiheitsgraden, nämlich unterschiedlichen Messlängen zwischen den Spannungselektroden und auch stromführenden Elektrodenpaaren läßt sich die tatsächlich elektrisch partizipierende "operative Thoraxlänge" bzw. das tatsächlich partizipierende elektrische "operative" Thoraxvolumen bzw. der tatsächlich gemessene "operative spezifische Widerstand" des Blutes mit Lösungen von Gleichungen mit mehreren Unbekannten elektrisch ermitteln oder auch mit empirisch gewonnenen Gleichungen dieses zusätzlich gewonnene Wissen in einem sogenannten "black box" Modell einbringen. So gehen nur mehr elektrisch gemessene Größen in diese Formel ein. Das Problem der Körpergröße, dass nämlich das Schlagvolumen und andere haemodynamischer Parameter nur beim Gesunden auf Grund der anthropometrischen Vorabinformation richtig bestimmt werden, wird damit eliminiert.

Eine mögliche weitere Ausgestaltung kann nun darin bestehen, dass die zumindest eine weitere Spannungs-Elektrode durch eine zweite Spannungs-Elektrode gebildet ist, die in einem bekannten, konstanten oder berechenbaren Abstand (d) zur ersten Spannungs-Elektrode angeordnet ist.

Damit kann die Messspannung gegenüber einer Bezugs-Spannungselektrode, die in einem anderen Bereich des Körpers angebracht ist, einerseits an der ersten Spannungs-Elektrode und andererseits an der zweiten Spannungs-Elektrode abgegriffen und aus den dadurch gewonnenen Messwerten bei bekanntem Abstand d zwischen der ersten und der zweiten Spannungs-Elektrode eine operative Mess-Länge zur Bezugs-Spannungselektrode für die Impedanzwertbestimmung ermittelt werden.

Der Abstand d zwischen der ersten und der zweiten Spannungs-Elektrode ist durch die Formgebung des erfindungsgemäßen Elektroden-Elements bekannt, es hat sich jedoch als eventuell zusätzlich günstig herausgestellt, ebenso wie bei der operativen Mess-Länge einen operativen Abstand zwischen der ersten und der zweiten Spannungs-Elektrode zu bestimmen.

Dies kann gemäß einer weiteren Ausführungsform der Erfindung dadurch erreicht werden, dass die zumindest eine weitere Spannungs-Elektrode durch die zweite Spannungs-Elektrode und eine dritte Spannungs-Elektrode gebildet ist, wobei die dritte Spannungs-Elektrode in einem Abstand zur ersten Spannungs-Elektrode angeordnet ist.

Bei Verwendung von Bandelektroden hat es sich herausgestellt, dass ein sehr hohes Maß an Reproduzierbarkeit der mit dem erfindungsgemäßen Elektroden-Element gemessenen Werte gegeben ist, wenn das Verhältnis der Streifenlänge zum Elektrodenabstand im Bereich zwischen ca.2, bevorzugt 4 und ca. 15, bevorzugt 10 liegt.

Dabei kann der Abstand zwischen den einzelnen Elektroden gemäß einer weiteren Ausbildung der Erfindung dadurch konstant gehalten werden, dass die erste Spannungs-Elektrode und die erste Strom-Elektrode sowie die zumindest eine weitere Spannungs-Elektrode und/oder die zumindest eine weitere Strom-Elektrode auf einem gemeinsamen elektrisch isolierenden Trägermaterial angeordnet sind.

Das Trägermaterial kann durch eine Trägerfolie gebildet sein, wobei die erste Spannungs-Elektrode und die erste Strom-Elektrode sowie die zumindest eine weitere Spannungs-Elektrode und/oder die zumindest eine weitere Strom-Elektrode auf einer Seite der Trägerfolie auf diese aufgebracht und vorzugsweise mit einer elektrisch leitenden Klebstoffschicht versehen sind. Auf diese Weise ist während der Durchführung des erfindungsgemäßen Verfahrens eine konstante Beabstandung der einzelnen Elektroden auf der Körperoberfläche gewährleistet.

Eine andere Variante der Erfindung kann darin bestehen, dass das Trägermaterial mehrere Folienstreifen mit einer klebfähigen Oberfläche umfasst, auf welche die erste Spannungs-Elektrode und die erste Strom-Elektrode sowie die zumindest eine weitere Spannungs-Elektrode und/oder die zumindest eine weitere Strom-Elektrode aufgebracht sind, und dass die Folienstreifen mit den darauf befindlichen Elektroden in im Wesentlichen paralleler Anordnung auf einer gemeinsamen Basis-Trägerfolie haften, welche Basis-Trägerfolie nach Aufbringen der Folienstreifen auf die Körperoberfläche von diesen abziehbar ist. Nach Abziehen der Basis-Trägerfolie verbleiben nur mehr die Folienstreifen mit jeweils einer Elektrode auf dem Körper des Patienten haften, die in elektrischem Kontakt mit der Körperoberfläche stehen. Während dadurch eine konstante Beabstandung zwischen den einzelnen Elektroden bestehen bleibt, ergibt sich aufgrund der geringen Gesamt-Kontaktfläche eine deutliche Reduktion von Haut-Irritationen, weshalb die Elektroden länger in Kontakt mit dem Patienten belassen werden können.

Um eine möglichst zuverlässige und leicht zu bedienende Verbindung mit den zum Betrieb des erfindungsgemäßen Elektrodenelements erforderlichen Anschlusskabeln zu erzielen, kann gemäß einer weiteren Ausführungsform der Erfindung die Trägerfolie an einem Längsende sich auf einer Seite zu einer Steck-Anschlussfläche verjüngen, auf der die erste Spannungs-Elektrode und die erste Strom-Elektrode sowie die zumindest eine weitere Spannungs-Elektrode und/oder die zumindest eine weitere Strom-Elektrode eng beabstandet geführt sind.

Eine andere Variante der Erfindung kann darin bestehen, dass die erste Spannungs-Elektrode und die erste Strom-Elektrode sowie die zumindest eine weitere Spannungs-Elektrode und/oder die zumindest eine weitere Strom-Elektrode in Form von Spot-Elektroden ausgeführt sind, die durch Abstandhalter, z.B. ebenfalls eine Trägerfolie oder durch ein gespanntes Band oder Kabel oder auch steife Abstandhalter voneinander beabstandet angeordnet sind. Auf diese Weise kann die Messung der Impedanz auch über sehr kleine Kontaktflächen auf der Körperoberfläche erfolgen.

Weiters betrifft die Erfindung ein Messsystem zur Messung der elektrischen Impedanz bzw. deren zeitliche Änderung an einem menschlichen Körper, insbesondere für eine Impedanz-Kardiographie (IKG)-Messung zur Bestimmung haemodynamischer Parameter, mit einer Mess-Wechsel-Stromquelle und einer ersten Spannungs-Messvorrichtung sowie einem erfindungsgemäßen medizinischen Elektroden-Element, wobei die Mess-Stromquelle mit der Strom-Elektrode und die Spannungs-Messvorrichtung mit der ersten Spannungs-Elektrode des Elektroden-Elements verbindbar sind.

Bei bisher verwendeten Messystemen zur Messung der elektrischen Impedanz im Körper musste die Messlänge zwischen zwei für die Impedanzmessung vorgesehenen Elektroden-Elementen oder zumindest die Körpergröße des Patienten, z.B. mit Hilfe eines Maßbandes, vermessen werden. Durch Messungenauigkeiten und aufgrund der Tatsache, dass die elektrisch tatsächlich wirkende Messlänge sich von der auf der Körperoberfläche zu messenden Beabstandung zwischen den jeweiligen Elektroden sehr, und in unvorhersehbarer Weise abweicht, kommt es zu Verfälschungen oder Ungenaugkeiten bei der Bestimmung des Messergebnisses.

Aufgabe der Erfindung ist es daher, ein Messsystem der vorstehend genannten Art anzugeben, mit dem die vorgenannten Probleme vermieden werden können.

Erfindungsgemäß wird dies dadurch erreicht, dass ein Umschalter vorgesehen ist, über den die Spannungs-Messvorrichtung entweder mit der ersten Spannungs-Elektrode oder mit der zumindest einen weiteren Spannungs-Elektrode verbindbar ist. Alternativ kann das Messsystem auch so aufgebaut sein, dass die Messung über verschiedene Messlängen gleichzeitig erfolgen kann.

Aus dem bekannten Abstand zwischen der ersten und der weiteren Spannungs-Elektrode kann die tatsächlich gegenüber einer Bezugs-Elektrode wirksame Messlänge zur Bestimmung der Impedanz aus dem eingeprägten Strom und den abgegriffenen Spannungswerten ermittelt werden.

Da das Ergebnis der Impedanzmessung am Körper des Patienten frequenzabhängig ist, kann eine Weiterbildung der Erfindung darin bestehen, dass die Mess-Wechsel-Stromquelle eine, eventuell auch kontinuierlich, veränderbare Messfrequenz aufweist. Auf diese Weise kann der Einfluss der Körperbestandteile, wie Blut, Gewebe und Knochen, auf das Messergebnis bei unterschiedlichen Messfrequenzen bestimmt werden.

In diesem Zusammenhang kann ein Phasendetektor zur Bestimmung des Phasenwinkels zwischen Messstrom der Mess-Stromquelle und der von der Spannungs-Messvorrichtung gemessenen Messspannung vorgesehen sein, sodass mit dem Phasenwinkel eine weitere Messgröße gewonnen werden kann.

Da die verschiedenen, durch das erfindungsgemäße Messsystem messbaren Impedanzwerte von der Lage des menschlichen Körpers im Raum abhängen, ist es vorteilhaft, den Winkel zwischen der Körperlängsachse und der Horizontalen oder Vertikalen aufzuzeichnen. Eine weitere Ausbildung des erfindungsgemäßen Messsystems kann daher darin bestehen, dass ein Winkelmesser zur Messung der Körperneigung vorgesehen ist. Dieser kann vorzugsweise in einem Verteilerstück angeordnet sein.

Die Messelektroden des erfindungsgemäßen Messsystems können auch für andere Zwecke verwendet werden, wobei es insbesondere als vorteilhaft erscheint, wenn die Elektroden-Elemente gemäß einer weiteren Ausführungsform der Erfindung mit einer EKG-Messvorrichtung verbunden sind.

Da das hier beschriebene, erfindungsgemäße Elektroden-Element und das damit durchführbare erfindungsgemäße Verfahren keine großen Ähnlichkeiten zur bisherigen Impedanzkardiographie aufweisen, wird für das hier beschriebene erfindungsgemäße Verfahren und Messsystem der neue Begriff "Multi-Site-Frequency Electromechanocardiographie (msf-ELMEC)" vorgeschlagen, das alle elektrisch bestimmbaren Parameter der Herzleistung wie z.B. Schlagvolumen, Inotropie, Auswurffraktion, diastolische Herzfunktion, Klappenveränderungen und potentiell andere haemodynamische Parameter, wie z.B. Pulmonalisdruck und weitere wichtige Parameter wie Volumen, die Verteilung, Zusammensetzung verschiedener Körperkompartimente bestimmen lässt.

Nachfolgend wird die Erfindung anhand der in den beigeschlossenen Zeichnungen dargestellten Ausführungsformen eingehend erläutert. Es zeigt dabei

Fig.3 ein Diagramm, das den Zusammenhang zwischen der am Körper gemessenen Messlänge und der ermittelten operativen Messlänge zwischen zwei Spannungs- oder Strom-Elektroden wiedergibt;

Fig.4A ein Diagramm, aus dem ein Vergleich der Herzschlagvolumensbestimmungen nach der herkömmlichen Impedanzkardiographie- und der Rebreathing-Methode hervorgeht;

Fig.4B ein Diagramm, aus dem ein Vergleich der Herzschlagvolumensbestimmungen nach der erfindungsgemäßen Impedanzkardiographie (msf-ELMEC)- und der Rebreathing-Methode hervorgeht;

Fig.5 zeigt ein nach der Zeit abgeleitetes Impedanzsignal, ein Elektrokardiogramm und und ein Phonokardiogramm eines Patienten;

Fig.6 ein nach der Zeit abgeleitetes Impedanzsignal für einen gesunden und einen erkrankten Patienten im Vergleich;

Fig.7 eine Ausführungsform des erfindungsgemäßen Elektroden-Elements;

Fig.8 eine weitere Ausführungsform des erfindungsgemäßen Elektroden-Elements;

Fig.9 eine schematische Darstellung einer Ausführungsform des erfindungsgemäßen Messsystems;

Fig.10 eine weitere schematische Darstellung einer Ausführungsform des erfindungsgemäßen Messsystems;

Fig.10a ein Detail einer Weiterbildung des erfindungsgemäßen Messsystems nach Fig.10;

Fig.10b ein Detail einer Weiterbildung des erfindungsgemäßen Messsystems nach Fig.10;

Fig.11 eine schematische Darstellung einer weiteren Ausführungsform des erfindungsgemäßen Messsystems;

Fig.12 eine schematische Darstelllung einer weiteren Ausführungsform des erfindungsgemäßen Messsystems und

Fig.13 eine schematische Darstellung einer weiteren Ausführungsform des erfindungsgemäßen Messsystems.

Beim erfindungsgemäßen Verfahren zur Messung des Volumens, der Zusammensetzung und Bewegung von elektrisch leitenden Körperflüssigkeiten, beruhend auf der elektrischen Impedanz des Körpers oder eines Körpersegments, insbesondere für die Elektromechanocardiographie (ELMEC)- bzw. Impedanz-Kardiographie (IKG)-Messung zur Bestimmung haemodynamischer Parameter, wird ein Wechsel-Messstrom zumindest einer Frequenz in den Körper eingebracht, und die Impedanz und deren Änderung über die Zeit des vom Wechsel-Messstrom durchflossenen, im wesentlichen selben Körpersegmentes bei zumindest zwei unterschiedlichen Messlängen im wesentlichen in Längsrichtung des Körpers gemessen.

Insbesondere für eine Impedanz-Kardiographie (IKG)-Messung zur Bestimmung haemodynamischer Parameter, z.B. des Herzschlagvolumens, wird über Strom-Elektroden, die auf der Körperoberfläche durch eine Stromelektroden-Messlänge voneinander beabstandet sind, ein Wechsel-Messstrom eingeprägt und über Spannungs-Elektroden, die auf der Körperoberfläche, insbesondere auf der Thoraxoberfläche, durch eine Spannungselektroden-Messlänge voneinander beabstandet sind, eine durch den Messstrom hervorgerufene Messspannung abgegriffen.

Aus dem Messstrom und der Messspannung wird die elektrische Impedanz bzw. deren zeitliche Änderung berechnet.

Beim erfindungsgemäßen Verfahren wird z.B. die Messspannung bei unterschiedlichen Spannungselektroden-Messlängen zwischen den Spannungs-Elektroden am im wesentlichen jeweils identen Körpersegment oder Segmenten bestimmt. Aus den sich ergebenden Messspannungswerten werden die zugehörigen Impedanzwerte berechnet. Die Veränderung der Messlänge erfolgt vorwiegend in Längsrichtung des Körpers und damit in Hauptrichtung des in diesem beförderten Blutes.

Weiters ist es vorteilhaft, bei zumindest zwei verschiedenen Messfrequenzen, besser zumindest drei bis 4 Frequenzen die Impedanz oder deren Änderung zu ermitteln. Dies setzt voraus, dass die zur Messung verwendete Mess-Wechsel-Stromquelle eine variable, eventuell auch kontinuierliche, veränderbare Messfrequenz aufweist.

Die Messfrequenzen sollten dabei soweit auseinander liegen, dass eine messbare Veränderung der Impedanz von Blut beobachtet werden kann, bzw. dass bei der höheren Frequenz ein Durchdringen der Körpermembranen durch den elektrischen Strom stattfindet. Als relevante Frequenzen werden z.B. zwischen 1 und 10 kHz, zwischen 30 und 100kHz, insbesondere ca. 40 kHz, und größer als 200 kHz, z.B. ca. 300 kHz bis 1 MHz genannt, wobei dies nur grobe Richtlinien für den gewünschten Frequenzumfang darstellt. Weiters wird vorgeschlagen eventuell statt der Messung von einzelnen Frequenzen ein "Frequenz-Sweep" über das gesamte in Frage kommende Frequenzspektrum, von einer unteren Messfrequenz bis zu einer oberen Messfrequenz, z.B. zwischen ca. 1 kHz und ca. 1000 kHz bzw. über einen in diesem Bereich liegenden interessierenden Abschnitt durchzuführen. Um das Signal-Rausch-Verhältnis der Messungen günstig zu halten, kann es sich als günstig erweisen bei mehreren Längen und mehreren Frequenzen alternierend oder gleichzeitig zu messen. Weiters kann es sich als günstig erweisen, mit einem speziellen Aufbau der Elektronik den Phasenwinkel des Impedanzsignals mitzubestimmen. Zu diesem Zweck ist ein Phasendetektor zur Bestimmung des Phasenwinkels zwischen Messstrom der Mess-Stromquelle und der von der Spannungs-Messvorrichtung gemessenen Messspannung vorgesehen.

Bekanntlich ist entsprechend der Definition des spezifischen Widerstandes die Impedanz:

$$Z_0 = \rho * ( L/A) \qquad (7)$$

wenn p der spezifische Widerstand der Messstrecke, L die Messlänge und A der Querschnitt der Messstrecke ist.

Ist nun eine zweite Messstrecke mit der Messlänge

$$L_2 = L + d \qquad (8)$$

vorhanden, wobei d ein konstanter oder berechenbarer Abstand zwischen beiden Messlängen L und $L_2$ ist, dann lässt sich sehr leicht ableiten, dass die operative elektrische Messlänge $L_o$ nach folgender Formel errechnet werden kann:

$$L_o = \frac{d}{\dfrac{Z_{02}}{Z_{01}} - 1} \qquad (9)$$

Setzt man nun diese elektrisch gemessene Messlänge $L_o$ z.B. in die KuBICEK-Gleichung ein, sind nun alle unbekannten Längenmaße aus der Formel verschwunden mit Ausnahme der genau durch die Formgebung der Elektroden definierten Differenz d (Elektrodenabstand).

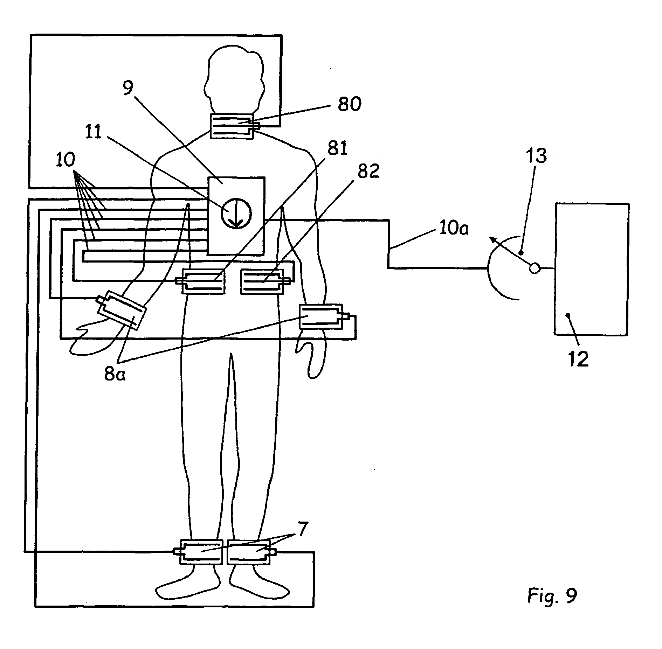

Bei einem Gerät zur Messung der elektrischen Impedanz bzw. deren zeitliche Änderung an einem menschlichen Körper zur Bestimmung von Körperflüssigkeiten, deren Zusammensetzung und deren Verschiebung im Körper, z.B. haemodynamischer Parameter sind erfindungsgemäß zwei Spannungselektroden 8a, 80 und 81, 82, 80', 81', 82', 85, 85', 85", 7 vorgesehen, von denen zumindest eine der beiden als Doppelspannungs-Elektrodenelement 80, 81, 82, 81', 82', 85, 85', 85" ausgeführt ist, wobei die Impedanz und deren Änderung nach der Zeit zwischen den zwei Spannungselektroden abgreifbar ist. In Fig. 9 ist die eine Spannungselektrode durch ein Dreifach-Elektrodenelement 80 und die zweite Spannungselektrode durch zwei auf gleicher Höhe angebrachte Dreifach-Elektrodenelemente 81, 82 gebildet.

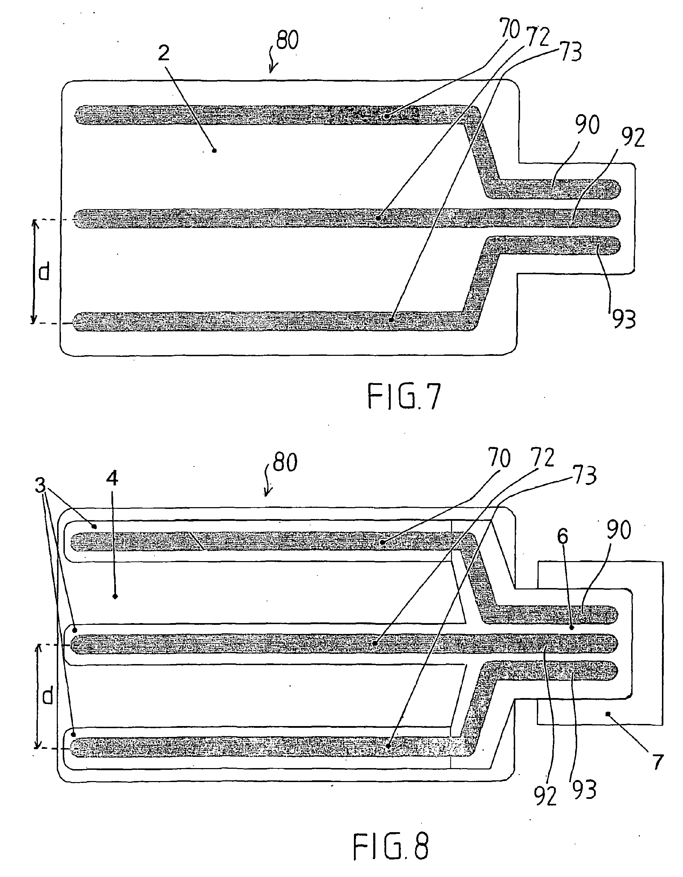

Der Aufbau der Dreifach-Elektrodenelemente 80, 81, 82, nämlich aus einer Strom-Elektrode 70 und zwei Spannungs-Elektroden 72, 73, die ein Doppelspannungs-Elektrodenelement bilden, ist in Fig.7 dargestellt, wobei die Strom-Elektrode 70 und die zwei Spannungselektroden 72, 73 in konstantem und bekanntem Abstand zueinander, am besten zur Wahrung des konstanten Abstandes auf einer gemeinsamen Trägerfolie 2, angebracht sind.

Die Differenz $d$ ist zwar durch die Formgebung der Elektroden 72, 73 bekannt, sie kann allerdings genauso elektrisch ungültig sein, wie die an der Thoraxoberfläche gemessene Länge $L$. Deswegen kann es sich als zusätzlich günstig erweisen, durch entsprechende Beschaltung der vorhandenen Messelektroden in Analogie zur Berechnung von $L_o$ nach Formel (9) auch ein operatives $d_o$ zu errechnen. Da es günstig ist, am an sich inhomogenen Stromfeld innerhalb des Thorax bei diesen Berechnungen von operativen Längen keine Eingriffe vorzunehmen, ist es günstig, die stromführenden Elektroden unverändert zu belassen und nur zusätzliche Messelektroden zu verwenden.

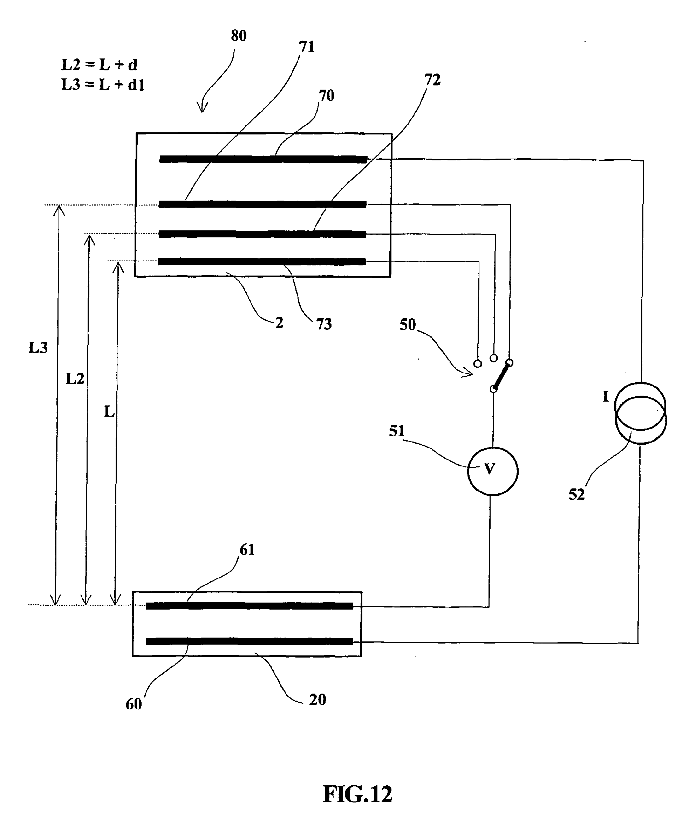

Die Berechnung eines $d_o$ macht z.B. das Anbringen einer zusätzlichen Messelektrode 71 bei einem erfindungsgemäßen Elektroden-Element 80 notwendig, wie es in Fig.12 gezeigt ist, sodass an der oberen Thoraxapertur dann ein Vierfachelektroden-Element, nämlich eine Strom-Elektrode 70 und drei Spannungs-Elektroden 71, 72, 73 angeordnet sind. Eingeprägt wird der Messstrom dabei über eine Mess-Wechsel-Stromquelle 52, die zwischen die Strom-Elektrode 70 und einer Strom-Elektrode 60 eines Elektroden-Elements 20 geschaltet ist, das z.B. an der unteren Thorax-Apertur aufgebracht ist. Wenn der Strom z.B. am unteren Ende des Körpers des Patienten 7 oder am unteren Ende des Rumpfes 85, 85' eingeprägt wird, genügt auch ein Dreielektrodenelement mit drei Spannungselektroden, das wahlweise entweder an der unteren oder oberen Thoraxapertur angebracht sein kann.

Eine Spannungs-Messvorrichtung 51 ist einerseits mit einer Spannungs-Elektrode 61 des Elektroden-Elements 20 verbunden und über einen Umschalter 50 entweder mit der ersten Spannungs-Elektrode 73 oder mit einer weiteren, nämlich der zweiten und der dritten Spannungs-Elektrode 72, 71 verbindbar. Die Messlänge zwischen der Spannungs-Elektrode 61 und der ersten Spannungs-Elektrode 73 beträgt L, die Messlänge zwischen der Spannungs-Elektrode 61 und der zweiten Spannungs-Elektrode 72 beträgt L2 und die Messlänge zwischen der Spannungs-Elektrode 61 und der dritten Spannungs-Elektrode 71 ist schließlich gleich L3. Es gilt ferner L2 = L + d und L3 = L + d1.

Durch Steuerung des Umschalters 50 können insgesamt drei Messspannungen abgegriffen werden, die den Messlängen L, L2 und L3 entsprechen.

Nach Berechnung von $d_o$ kann dieser Wert dann statt d in die Gleichung (9) eingesetzt werden.

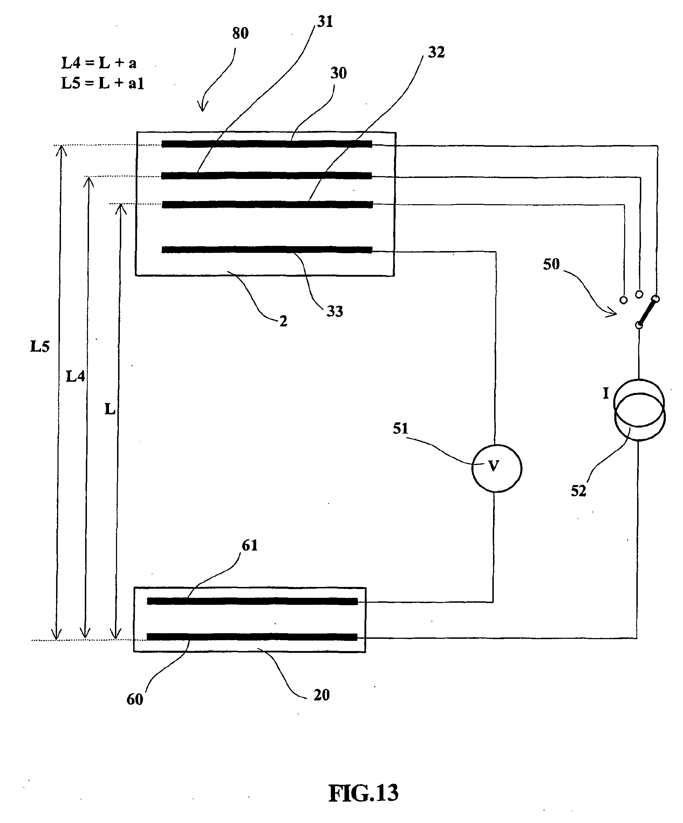

Eine Variante des erfindungsgemäßen Messsystems, bei dem anstelle von mehreren Spannungs-Elektroden mehrere Strom-Elektroden vorgesehen sind, ist in Fig.13 gezeigt.

Ausgangspunkt ist wiederum ein Vierfachelektrodenelement 80, auf dem eine Spannungs-Elektrode 33 und drei in einem definierten Abstand zueinander angeordnete Strom-Elektroden 30, 31, 32 vorgesehen sind. Die Messlänge zwischen der Strom-Elektrode 60 des Elektroden-Elements 20 und der Strom-Elektrode 32 beträgt L, sie erhöht sich auf L4 für die Strom-Elektrode 31 und auf L5 für die Strom-Elektrode 30, wobei L4 = L + a und L5 = L + a1 ist.

Der Messstrom wird über die Mess-Wechsel-Stromquelle 52, die einerseits an die Strom-Elektrode 60 des Elektroden-Elements 20 angeschlossen und andererseits über den Umschalter 50 an die Strom-Elektroden 30, 31, 32 schaltbar ist, eingeprägt.

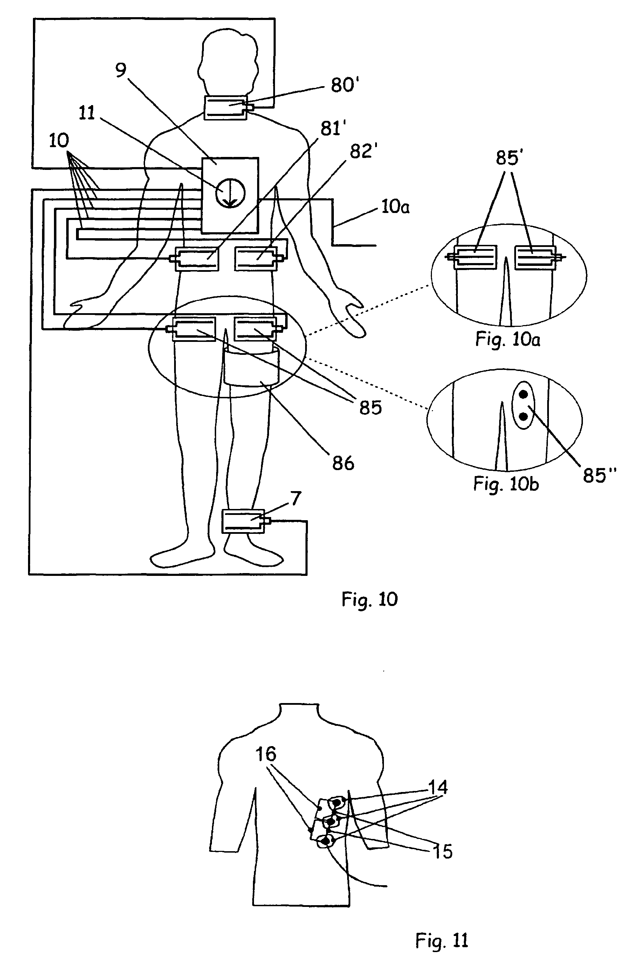

Die Spannungs-Messvorrichtung 51 ist mit der Spannungs-Elektrode 61 des Elektroden-Elements 20 verbunden und mit der Spannungs-Elektrode 33 verbunden, sodass durch Spannungsmessung bei drei verschiedenen Messlängen L, L4 und L5 der Stromeinprägung die Impedanz und eine operative Meßlänge ermittelt werden kann, die sich aus analogen Überlegungen zum Ausführungsbeispiel gemäß Fig.11 ergibt.

Fig. 3 zeigt die in praktischen Versuchen ermittelten Unterschiede zwischen der tatsächlichen, an der Körperoberfläche gemessenen Länge in cm und die aus dem Ohm'schen Gesetz errechnete "operativen Länge" $L_o$ für ein Elektroden-Element gemäß Stand der Technik. Wie ersichtlich ist der Zusammenhang beider Längen äußerst unbefriedigend, was darauf hinweist, dass zwischen der an der Körperoberfläche gemessenen Länge und der operativen Länge, wie sie sich aus der oben angeführten Formel ergibt, kein klinisch relevanter Zusammenhang besteht. Vor allem kann man in der Fig. 3 beobachten, dass die gemessene Länge wesentlich länger ist als die virtuelle Länge, was darauf hinweist, dass zwischen den Elektroden wesentliche Änderungen des Durchmessers des elektrisch partizipierenden Thoraxgewebes bestehen müssen, die offensichtlich als "elektrische Bäuche" den virtuellen Elektrodenabstand verkürzen, und das in nicht vorherzusehender Weise.

Man sieht also, dass bisher bei der Impedanzkardiographie von völlig falschen theoretischen Überlegungen ausgegangen wurde. Dies ist sehr leicht erklärlich, da die Elektrizitätsverteilung im Thorax äußerst inhomogen ist, und die Leitung durch die unterschiedlichen Medien wie Haut, Knochen, Fett, Lunge, Herz und Gefäße sich unterschiedlich verteilt. Bisher war die Reproduzierbarkeit der Impedanzkardiographie durch diese Inhomogenitäten auch sehr begrenzt. Um halbwegs reproduzierbar zu sein, mussten die Elektroden möglichst genau wieder an dieselbe Stelle am Thorax plaziert werden. Dies ist zwar im Kurzzeitversuch möglich, nicht jedoch bei Langzeitbeobachtungen über Tage oder länger. Mit dem erfindungsgemäßen Verfahren ist diese unabhängig vom Aufbringungsort der Elektroden geworden, weil sich die Methode ja automatisch immer durch Messung der operativen Messlänge korrigiert, auch wenn sich die Verhältnisse innerhalb des Thorax, z.B. durch eine unterschiedliche Flüssigkeitsverteilung geändert haben sollten.

Eine sehr vorteilhafte Methode stellt auch das Anbringen der stromführenden Elektrode an den Extremitäten 7, 8a dar, weil von diesen der Strom hauptsächlich entlang der großen Gefäße und entlang der Aorta erfolgt. Der Messstrom wird dabei über zwei Strom-Elektroden an jeweils zumindest einer Körperextremität, z.B. an einem Bein und/oder an einem Arm eingeprägt.

Damit ergibt sich im Thorax ein weit homogeneres Stromfeld, als wenn die stromführende Elektrode direkt am Thorax angebracht ist. Damit würde an der unteren Thoraxapertur das Anbringen einer Doppelelektrode genügen, wie sie z.B. in der Patentschrift A392/2001 beschrieben ist. Bei Anbringen von zwei Doppelelektroden, z.B. der in A392/2001 beschriebenen Elektrode oder zwei benachbarten Einzelelektroden am oberen Körperende (z.B. Halsgegend, Kopf oder Arme) bzw. am unteren Körperende kann dann gleichzeitig auch die Körperflüssigkeit gemessen werden, indem nämlich die Ganzkörperimpedanz mitgemessen wird. Dies ist deswegen wichtig, da der Flüssigkeitsgehalt des Thorax in Beziehung zum Flüssigkeitshaushalt des Organismus gesehen werden muss. Gerade bei Herzinsuffizienz ist die Flüssigkeitsverteilung im Thorax in Beziehung zur Gesamtkörperflüssigkeit sehr gestört, was die Anwendung der Impedanzkardiographie bei Herzinsuffizienz bisher unmöglich gemacht hat.

Ein weiteres Hauptproblem der Impedanzkardiographie ist die Miteinbeziehung des spezifischen Blutwiderstandes, der quantitativ in die Formel eingehen sollte. Deswegen wird zumindest in der KUBICEK-Gleichung der aus dem Hämatokrit berechnete spezifische Blutwiderstand in die Formel eingebracht. QUAIL ET AL. [xi] schrieben die KUBICEK-Gleichung um und errechneten den spezifischen Widerstand aus dem Schlagvolumen und anderer haemodynamischer Parameter, das sie mit Hilfe eines elektromagnetischen Flussmessgerätes (EMF) bei Hunden bestimmten:

$$\rho_{Blut} = \frac{SV_{EMF} \cdot Z_0^2}{l^2 \cdot LVET \cdot (dZ/dt)_{max}} \qquad (10)$$

QUAIL ET AL. fanden heraus, dass $\rho_{Blut}$ zwar vom Hämatokrit abhängig ist, aber ansonsten näherungsweise konstant bleibt. Sie ersetzten $\rho_{Blut}$ durch einen mittleren thorakalen Widerstand $\rho_0$. Wenn $\rho_0$ näherungsweise eine Konstante in KUBICEK's Gleichung ist, so kann man es wie folgt ersetzen:

$$Z = \rho \cdot \frac{l}{A} \Rightarrow \rho_0 = \frac{Z_0 \cdot A}{l} \qquad (7 => 11)$$

Nun setzt man in KUBICEK's Gleichung ein:

$$SV = \frac{Z_0 \cdot A}{l} \cdot \frac{l^2}{Z_0^2} \cdot LVET \cdot (dZ/dt)_{max}$$

$$SV = \frac{A \cdot l}{Z_0} \cdot LVET \cdot (dZ/dt)_{max} \qquad (12a, b, c)$$

$$SV = V_{Thorax} \cdot LVET \cdot \frac{(dZ/dt)_{max}}{Z_0}$$

Wir haben nun den sehr schwer bestimmbaren spezifischen Widerstand (Resistivität p) aus der Gleichung für das Schlagvolumen und anderer haemodynamischer Parameter mathematisch eliminiert. Die Voraussetzung dafür ist, dass dieser spezifische Blutwiderstand während der Herztätigkeit konstant bleibt. Nach SHANKAR ET AL. [xii] ist die Änderung der Resistivität in Abhängigkeit von der Aktivität des Herzens kleiner als 5.5% und somit ist die Gleichung hinreichend genau.

Eine Alternative würde darstellen, dass man p zu unterschiedlichen Zeitpunkten der Herztätigkeit mißt, nämlich z.B. zur Zeit des maximalen Blutflusses, während der Systole, sowie auch zur Zeit des minimalen Blutflusses, am Ende der Diastole. Besser ist es, wie auch bei der Impedanzkardiographie üblich, die Änderung des Impedanz nach der Zeit $(dZ/dt)_{max}$ zu beobachten, da dieser Wert von der Herzaktion und somit von der Blutmenge aus dem Herzen (SV) determiniert ist. Für die neue Methode muss nur die Eigenschaft benützt werden, dass der spezifische Widerstand des Blutes p, speziell der roten Blutkörperchen (Erythrocyten), bei unterschiedlichen Frequenzen des Wechselstroms unterschiedlich ist. So ist z.B. der elektrische Widerstand des Blutes bei 20 kHz deutlich höher als beispielsweise bei 100 kHz, wobei bei noch höheren Frequenzen die Leitfähigkeit der Erythrocyten noch weiter ansteigt, weil sich bei höheren Frequenzen die Erythrozytenmembran wie ein elektrischer Kondensator verhaltet. Diese Eigenschaft dann kann zur Bestimmung von p ausgenützt werden, genauer ist die Abnahme des $(dZ/dt)_{max}$ bei höheren Frequenzen ein Maß für die Anzahl der roten Blutkörperchen, also des mit dem Hematokrit in Verbindung stehenden spezifischen Widerstandes des Blutes. Je größer die Abnahme von $(dZ/dt)_{max}$ beim Wechsel von einer niedrigen zu einer hohen Frequenz, desto größer ist die Anzahl der roten Blutkörperchen, da ja das Schlagvolumen beim spezifischen Herzschlag gleich bleibt.

$$\rho = f(Hkt) = F\left( \frac{\left(\frac{dZ}{dt}\right)_{max\_freq1}}{\left(\frac{dZ}{dt}\right)_{max\_freq2}} \right) \qquad (13)$$

Genauso kann bei nichtlinearem Zusammenhang bei mehr als 2 Frequenzen gemessen werden und alle sich daraus möglicherweise ergebenden Funktionen auch als eine nichtlineare Regressionsgleichung ausgedrückt werden. Die Funktion F kann in beiden Fällen (2 Frequenzen, mehrere Frequenzen) durch Vergleichsmessungen mit der herkömmlichen Bestimmung des Hematokrites Hkt empirisch bestimmt werden.

Eine weitere Methode, aus elektrisch gemessenen Signalen auf die Änderung des Blutwiderstandes während des Herzzykluses zu kommen ergibt sich ebenfalls aus der oben genannten Methode: WANG ET. AL.[xiii] haben gezeigt, dass die Änderung der Blut-Resistivität ⁻25% der Impedanzänderung ausmacht, also ein bedeutendes Maß für die Berechnung des richtigen SV ist. Diese Blut-Resistivitätsänderung ist nun ein direktes Maß aus dem Verhältnis zwischen den beiden differenzierten Impedanzsignalen gemessen bei unterschiedlichen Frequenzen $(dZ/dt)_{freq1}/(dZ/dt)_{freq2}$. So kann nun ein zusätzliches Signal $\Delta\rho(t) = (dZ/dt)_{freq1}/(dZ/dt)_{freq2}$ ständig gewonnen werden, das ein Maß für die Blut-Resistivitätsänderung während des Herzzyklusses darstellt. Dazu müsste nur z.B. ein gleitendes schmales Fenster über den Herzzyklus gelegt werden, um für jedes dieser schmalen Fenster das $\Delta\rho(t)$ zu bestimmen. Der maximale Ausschlag dieses Signales $\Delta\rho_{max}$ kann nun ebenso für die Bestimmung des HZV verwendet werden. So ist es nicht unbedingt notwendig, die Funktion F vorher empirisch zu bestimmen.

$$\Delta\rho(t) = \frac{(dZ/dt)_{freq1}}{(dZ/dt)_{freq2}} \qquad (14)$$

Ein zusätzlicher Vorteil der Methode ist der, dass bei genügend präziser Feststellung des $\Delta\rho(t)$-Signals nach obiger Methode nun auch damit festgestellt werden kann, ob das Blut fließt oder nicht und welcher Art die Strömung beschaffen ist. Bei laminarer Strömung nimmt nämlich der Widerstand durch Ausrichtung der Erythrozyten in der Strömungsrichtung ab, um bei höherer Geschwindigkeit wieder durch die Turbulenzen zuzunehmen. Aus der Formkurve der kontinuierlich gemessenen $\Delta\rho(t)$ - Werte kann dann festgestellt werden, wie lange das Blut fließt, und ob die Strömung des Blutes laminar oder turbulent ist. Damit könnte auch die LVET (linksventrikuläre ejection time), die konventionell aus dem IKG Signal direkt oder aus dem Phonokardiogramm bestimmt wird, durch eine Messung aus dem sich verändernden p bestimmt werden.

Die genaue Bestimmung der Blut-Resistivität p, die richtige operative Länge $L_0$, bzw. die Grundimpedanz $Z_o$, die ein Maß für die Thoraxgeometrie ist, ist für die Bestimmung des richtigen SV notwendig, denn aus diesen Größen läßt sich die richtige Bestimmung des elektrisch partizipierenden Thoraxvolumen $V_{Thorax}$ ableiten. Dieses elektrisch partizipierende Thoraxvolumen $V_{Thorax}$ ist das wichtige Maß für die Bestimmung des SV mit der Hilfe der Impedanzkardiographie, wie man aus der allgemeinen Gleichung des IKG (12c) ersehen kann:

$$SV = V_{Thorax} \cdot LVET \cdot \frac{(dZ/dt)_{max}}{Z_0} \qquad (12c)$$

Wie schon vorhin beschrieben berechnet KUBICEK dieses $V_{Thorax}$ aus dem Elektrodenabstand L, dem spezifischen Widerstand des Blutes p und der Grundimpedanz $Z_0$, SRAMEK oder BERNSTEIN schätzen weiters $V_{Thorax}$ aus der Körpergröße H und/oder dem Gewicht des Patienten W. Weitere "verbesserte" Formeln, die bei verschiedensten IKG-Geräten zum Einsatz kommen, verarbeiten sogar das Alter des Patienten, um $V_{Thorax}$ zu bestimmen.

Diese Werte, bzw. lineare Abhängigkeiten dieser Werte lassen sich aber nun, wie oben beschrieben, aus elektrisch messbaren Größen bestimmen. Somit ist auch das $V_{Thorax}$ einzig aus verschieden elektrischen Größen bestimmbar.

$$V_{Thorax} = f(L, \rho, Z_o, H, W)$$

$$V_{Thorax} = f\left( \frac{d}{\frac{Z_{02}}{Z_{01}} - 1}, \frac{\left(\frac{dZ}{dt}\right)_{max\_freq1}}{\left(\frac{dZ}{dt}\right)_{max\_freq2}}, \frac{Z_{freq1}}{Z_{freq2}}, \frac{Z_{freq1}}{Z_{freq3}}, \ldots \right) \qquad (15)$$

Diese nun ausschließlich elektrisch gemessenen Größen können nicht nur in jede bekannte Formel zur Bestimmung des Schlagvolumen und anderer haemodynamischer Parameters eingebracht werden, wie z.B. in die Formeln von KUBICEK, SRAMEK oder BERNSTEIN, sondern auch in jede beliebige Gleichung zur Ermittlung des Schlagvolumen und anderer haemodynamischer Parameters; dabei könnte es sich auch um nicht abgeleitete, sondern empirisch gewonnene Gleichungen handeln, die mittels Vergleich mit einem Goldstandard wie z.B. dem invasiven Fick'schen Prinzip, der Thermodilution oder der Atemgasmethode gewonnen werden. So bleiben nur mehr elektrisch gemessene Größen in der Gleichung, woraus sich eine sehr viel größere Präzision ergibt. Die Qualität des einzelnen Signals ist dabei von untergeordneter Bedeutung, da ja bei jedem Herzschlag, also ungefähr 70 mal in der Minute gemessen werden kann und die aus jedem einzelnen Herzschlag berechneten Größen entweder gemittelt werden oder aber ein genaues Template aus dem Impedanzsignal ermittelt wird.

In ähnlicher Weise können die elektrisch bestimmten Parameter L und p in jede bekannte oder neu zu entwikkelnde Gleichung zur Errechnung des Schlagvolumen und anderer haemodynamischer Parameters eingebracht werden. Auch können die so elektrisch ermittelten zahlreichen Parameter auch zur Berechnung von anderen wichtigen Parametern der mechanischen Herzfunktion, z.B. der Auswurffraktion, Kontraktilität, Inotropie, bzw. Pulmonalisdruck usw. verwendet werden.

Nach unseren Erfahrungen hat es sich zumindest so bewährt, empirische Formeln für Schlagvolumen und andere haemodynamische Parameter, wie z.B. Auswurffraktion, Inotropie usw. zu verwenden, die unter Verwendung des Goldstandards für die oben angeführten Größen erstellt werden. Dabei könnten die ermitteln Größen $Z_{01}$, $Z_{02}$, $(dZ/dt)_{max\_freq1}$, $(dZ/dt)_{max-freq2}$, $Z_{freq1}$, $Z_{freq2}$, $Z_{freq3}$, ..., vorzugsweise in einer multiplen Regressionsanalyse und/oder mit neuronalen Netzen und/oder mit weiteren Machine Learning"-Algorithmen mit dem tatsächlichen Schlagvolumen und anderer haemodynamischer Parameter, das durch eine Goldstandardmethode gewonnen wurde, in Beziehung gebracht werden. Als Goldstandard für das tatsächliche Schlagvolumen und anderer haemodynamischer Parameter würde sich selbstverständlich in erster Linie das Fick'sche Prinzip, die Thermodilution oder die Atemgasmethode eignen. Daraus könnte eine multiple Regressionsgleichung erstellt werden, die rein empirisch den besten Zusammenhang zwischen obigen Parametern und den tatsächlichen Schlagvolumina beschreibt, damit wären alle Inhomogenitäten der biologischen Messung, wie z.B. die Tatsache, dass es sich bei dem Thorax nicht um einen geometrisch genau definierten Körper handelt und dass die Homogenität der Elektrizitätsausbreitung im Thorax bei den unterschiedlichen Geweben, wie Fett, Muskel, Rippen, Haut, Lunge, Gefäßbänder und Herz, nie hinreichend genau mathematisch beschrieben werden kann. Wegen der Nichtlinearitäten kann sich auch eine multiple Polynomgleichung ergeben. Besonders wichtig ist bei diesen Formeln auch die Verwendung der Ganzkörperimpedanz, und dies bei mehreren Frequenzen, z.B. bei ungefähr 1-10 kHz, 40 kHz und 200 kHz und eventuell auch bei noch sehr viel höheren Frequenzen, weil damit das Körperwasser, der Extrazellulärraum und das intrazelluläre Wasser und ihre Relation zum Flüssigkeitsgehalt des Thorax berücksichtigt wird. In diese Formeln würden all die elektrisch gemessenen Größen eingehen, die in einer partiellen Korrelationsanalyse gezeigt haben, in signifikanter Beziehung zur mechanischen Leistung des Herzens zu stehen.

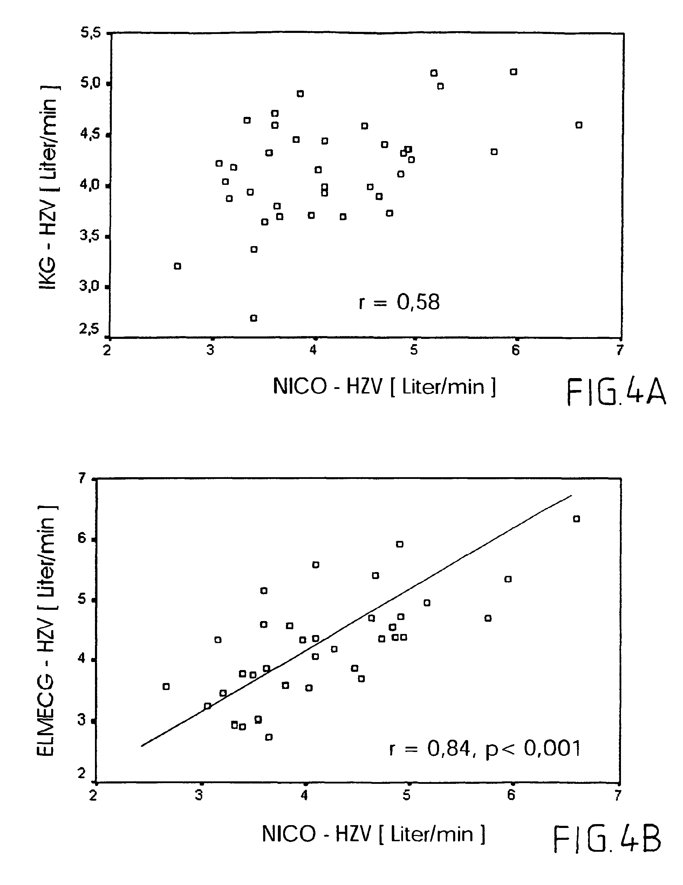

Fig. 4 zeigt beispielsweise eine mit Hilfe einer einfachen Applikation der hier vorgestellten Methode gewonnene Berechnung des HZV bei einem unselektionierten Krankengut, also auch Patienten mit Herzinsuffizienz, das wegen verschiedenster Krankheiten einer Operation zugeführt werden musste. Während der Operation wurde mittels des NICO Gerätes, das über $CO_2$ Rebreathing das HZV misst, und das bei beatmeten Patienten eine ausgezeichnete Übereinstimmung mit der Thermodilution aufweist, das HZV als "Goldstandard" gemessen. Im oberen Teil der Abbildung ist dabei auf der x-Achse die Korrelation des NICO-HZV mit der herkömmlichen Impedanzkardiographie gezeigt. Wie ersichtlich beträgt r= 0,58, was einer klinisch sehr schlechten und damit nicht brauchbaren Korrelation entspricht (obwohl die Körpermaße mitverwendet wurden und so eine mathematische Voraussage des HZV eingeflossen ist). Im unteren Teil der Abbildung hingegen ist der Vergleich des NICO-HZV mit einer sehr einfachen Ausführung der msf-ELMEC gezeigt, ohne jede Verwendung von eingegebenen Körperparametern wie Gewicht und Größe, bei der der Korrelationskoeffizient r= 0,84 beträgt, was bereits einer klinisch sehr brauchbaren Korrelation entspricht. Diese Korrelation lässt sich bei komplexerer technischer Ausführung noch wesentlich verbessern.

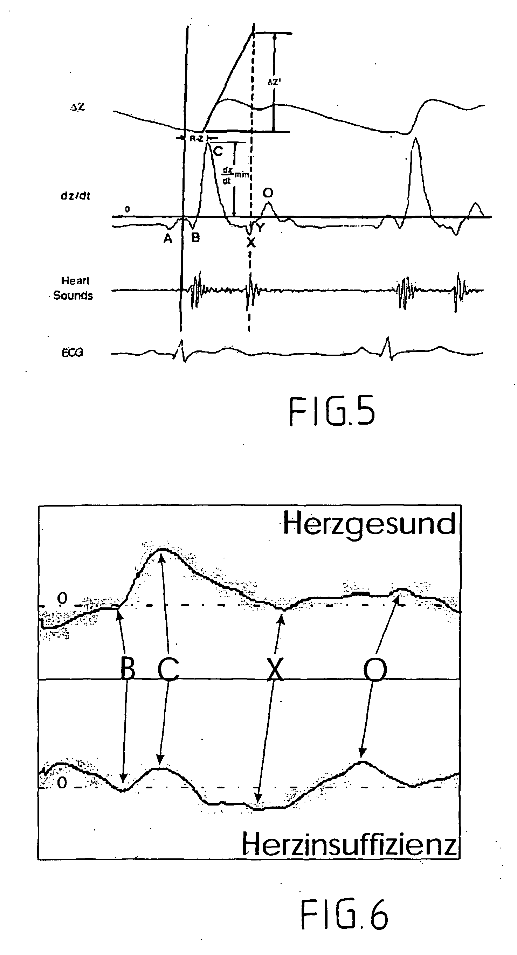

Fig. 5 zeigt das übliche differenzierte Impedanzsignal, mit der üblichen Nomenklatur, nämlich den Zeitpunkten A,B,C,X,Y und O, und dem gleichzeitig registriertem EKG und Phonocardiogramm. Die Zeiten des maximalen Blutflusses sind zwischen R-Z und die des minimalen Blutflusses unmittelbar vor der A Welle gegeben. Es ist dabei zu beachten, dass die Impedanzkurven ($\Delta Z$ und dZ/dt) konventionsgemäß im Vorzeichen umgedreht wird.

Eine weitere Möglichkeit die msf-ELMEC zu verbessern ergibt sich aus folgendem Ansatz:

Üblicherweise wird derzeit für die Auswertung der Impedanzkardiographie nur die Höhe des dz/dt verwendet obwohl in der Form des Impedanzsignals sehr viel mehr Information verborgen ist. So ändert sich bei Herzinsuffizienz die Form des Impedanzsignals wie in Fig. 6 gezeigt wird. Im oberen Teil der Fig. 6 ist ein dZ/dt-Signal eines Herzgesunden zu sehen, im unteren Teil das dZ/dt-Signal eines Patienten mit Herzinsuffizienz. Wie ersichtlich wird zwar bei Herzinsuffizienz das $(dZ/dt)_{max}$ (C-Punkt) kleiner, zusätzlich kommt es jedoch zu zusätzlichen Veränderungen, wie einer Zunahme der Amplitude der X-Welle und der O-Welle.

Wie in dieser Abbildung gezeigt, sollte eventuell zusätzlich statt des alleinigen $(dZ/dt)_{max}$ die Amplituden der negativen Welle B, der positiven Amplitude der Welle C (das eigentliche $(dZ/dt)_{max}$), der negativen Welle X und der positiven Welle O, weiters auch die dazugehörigen Anstiegs- und Abfalls-Steilheiten sowie Flächenintegrale in die Formel eingebracht werden.

Zusätzlich kann es eventuell notwendig werden, die Lage des menschlichen Körpers im Raum entlang der Längsachse zu wissen, da die verschiedenen $Z_0$ bei unterschiedlicher Lage auch unterschiedlich in die Gleichung eingehen können. Dazu kann es sich als günstig erweisen, die Lage des Körpers mitzubestimmen, zu diesem Zweck kann am Körper ein Winkelmesser angebracht sein, der vorteilshafter Weise z.B. im Verteilerstück untergebracht sein könnte, sodass er verborgen ist und auch bei jedem Patient wieder verwendet werden kann. In diesem Fall würden die Gleichungen zur Bestimmung von Schlagvolumen und anderer haemodynamischer Parameter für die unterschiedliche Körperlage korrigiert werden.

Mit all diesen Neuerungen scheint nun erstmals die Methode physikalisch genau genug, ausschließlich elektrisch definiert, von hoher Präzision und Reproduzierbarkeit und damit auch erstmals für die Diagnose von Herzkrankheiten und zur Überwachung im Intensiv- und Anästhesiebereich geeignet. In Gegensatz zu allen anderen Methoden müssen nur mehr wenige, angenehm zu tragende elektrische Elektroden auf den Brustkorb und an den Extremitäten aufgebracht werden, die gleichzeitig auch zur Ableitung des EKGs zur Verfügung stehen; es müssen nicht mehr am Thorax fragwürdige Längenmessungen durchgeführt werden, die durch die asymmetrische Form des Thorax und die vorhandenen Mehrfachelektroden mit notwendigen Mittelwertbildungen ja nie präzise sein können; es muss nicht mehr die Körpergröße des Patienten eingegeben werden, die ja, wie bereits ausgeführt, unerwünschtes, fehlerbegünstigendes Bias in die Gleichung einbringt; der Patient muss nicht mehr einen Katheter in die Pulmonalarterie oder in eine andere Arterie eingeführt bekommen und er muss nicht mehr durch ein Mundstück in einem geschlossenen System atmen. Auch die Applikation eines Farbstoffes, der transkutan gemessen wird, wird damit überflüssig.

Fig. 7 zeigt eine beispielsweise Ausprägung der dazu notwendigen, erfindungsgemäßen Elektroden-Elemente.

Auf diesem weist eine erste Strom-Elektrode 70 einen Strom-Anschluss 90 zum Einprägen eines elektrischen Wechsel-Messstromes auf, während bei einer von dieser beabstandeten, ersten Spannungs-Elektrode 73 ein Spannungs-Anschluss 93 zum Abgreifen einer elektrischen Messspannung ausgebildet ist.

Erfindungsgemäß ist eine weitere, hier eine zweite, Spannungs-Elektrode 72 mit einem Spannungs-Anschluss 92 vorgesehen, die in einem Abstand d zur ersten Spannungs-Elektrode 73 angeordnet ist. Im Rahmen der Erfindung können auch mehrere weitere Spannungs-Elektroden oder mehrere Strom-Elektroden vorgesehen sein.

Die erste Spannungs-Elektrode 73 und die erste Strom-Elektrode 70 sowie die zumindest eine weitere Spannungs-Elektrode 72 sind in Form von zueinander parallelen, elektrisch leitenden Streifen ausgebildet, die auf einem gemeinsamen elektrisch isolierenden Trägermaterial, insbesondere eine Trägerfolie 2 angeordnet sind. Es sind dabei die erste Spannungs-Elektrode 73 und die erste Strom-Elektrode 70 sowie die zweite Spannungs-Elektrode 72 auf einer Seite der Trägerfolie 2 auf diese aufgebracht und vorzugsweise mit einer elektrisch leitenden Klebstoffschicht versehen.

Zur Vereinfachung der Herstellung eines elektrischen Kontaktes mit einer Mess-Stromquelle bzw. mit einer Mess-Spannungsvorrichtung verjüngt sich weiters die Trägerfolie 2 an einem Längsende auf einer Seite zu einer Steck-Anschlussfläche 6, auf der die erste Spannungs-Elektrode 73 und die erste Strom-Elektrode 70 sowie die zweite Spannungs-Elektrode 72 eng beabstandet geführt sind. Die Trägerfolie 2 besteht vorzugsweise aus einem hautverträglichen, nicht leitenden, schmiegsamen Material und kann zwischen den Elektroden 70, 72, 73 durchgehend ausgeführt sein, wie das im Ausführungsbeispiel gemäß Fig.7 gezeigt ist.

Im Ausführungsbeispiel gemäß Fig.8 umfasst hingegen das Trägermaterial mehrere Folienstreifen 3 mit einer klebfähigen Oberfläche, auf welche die erste Spannungs-Elektrode 73 und die erste Strom-Elektrode 70 sowie die zumindest eine weitere Spannungs-Elektrode 72 aufgebracht sind, wobei die Folienstreifen 3 mit den darauf befindlichen Elektroden 70, 72, 73 in im Wesentlichen paralleler Anordnung auf einer gemeinsamen Basis-Trägerfolie 4 haften, welche Basis-Trägerfolie 4 nach Aufbringen der Folienstreifen 3 auf die Körperoberfläche von diesen abziehbar ist.

Der konstante Abstand zwischen den Elektroden 70, 72, 73 ist dabei dadurch gewährleistet sein, dass die Basis-Trägerfolie 4 nach deren Befestigung am Thorax wieder abgezogen wird. Dies hat den Vorteil, dass eine Hautreizung von der großen Fläche einer sehr breiten Trägerfolie 2 der Ausführungsform gemäß Fig.7 auf eine sehr kleine Fläche reduziert wird.

Das in Fig.8 gezeigte erfindungsgemäße Elektroden-Element 80 weist in bekannter Weise vorzugsweise auf der dem Körper zugewandten Seite anfänglich eine Abziehfolie auf, welche die leitende Beschichtung der Elektroden 70, 72, 73 und den nichtleitenden Klebstoff der Basis-Trägerfolie 4 feucht hält und die erst unmittelbar vor Verwendung abgezogen wird, wie das von allen medizinischen Elektroden hinreichend bekannt ist.

Natürlich könnten zusätzlich weitere Spannungs- und Strom-Elektroden auf dieser Trägerfolie 4 aufgebracht sein, z.B. auch eine zusätzliche Stromelektrode oder auch zusätzliche Spannungs-Elektroden, um die operative Elektroden-Messlänge $L_o$ bzw. den operativen Abstand $d_o$ noch genauer mathematisch zu errechnen. Die Verwendung einer gemeinsamen Strom-Elektrode 70 hat hingegen den Vorteil, dass das elektrische Feld im Thorax, sowohl bei Messung von $Z_{01}$, als auch $Z_{02}$, sich in seiner Inhomogenität nicht ändern kann.

Eine weitere Ausführungsform des erfindungsgemäßen Elektroden-Elements zum Gewährleisten des konstanten Abstand kann darin bestehen, dass eine zusätzliche Träger-Folie an der vom Körper abgewandten Seite angebracht ist, die erst nach Befestigung der fingerförmigen Trägerfolie am Thorax abgezogen wird. Die Träger-Träger Folie sollte daher auch auf der Fläche zwischen den Trägerfolien keinen hautreizenden Klebstoff aufweisen.

Wie aus der Fig. 8 weiters ersichtlich, werden die Elektroden 70, 72, 73 im Bereich einer Steckverbindung 7 für eine Impedanzmessvorrichtung vorzugsweise in einer seitlichen Verjüngung 6 der Folienstreifen 3 zusammengeführt, um so hier eine schmale, im klinischen Alltag praktikable und kostengünstige Steckverbindung 7 verwenden zu können.

Fig. 9 zeigt einen Messaufbau am Körper eines Patienten unter Einbeziehung des erfindungsgemäßen Messsystems. An den distalen Enden von Extremitäten, wie Armen und Beinen sind Elektroden-Elemente 7 und 8a angebracht, die zur Messung der Ganzkörperimpedanz am besten bei zwei, drei oder mehreren Frequenzen dienen und sowohl Strom- als auch Spannungs-Elektroden beinhalten. Um eine asymmetrische Stromverteilung innerhalb des Körpers zu vermeiden, erfolgt im gezeigten Ausführungsbeispiel die Anbringung der Elektroden-Elemente 7 und 8a an beiden Beinen und an beiden Armen, wobei der eingeprägte Strom in jeder Körperhälte gleich groß gewählt wird. Alternativ könnte über nur einen Arm und nur ein Bein ein Messstrom eingeleitet werden.

An der unteren Thoraxapertur sind weiters die zwei Dreifach-Elektrodenelemente 81, 82 und im Halsbereich das Dreifach-Elektrodenelement 80 mit jeweils einer Spannungs-Elektrode und zwei Strom-Elektroden angebracht. Über die Strom-Elektrode des oberen (Halsbereich) Dreifach-Elektrodenelements 80 und der Strom-Elektrode der unteren Elektrodenelemente 81 an der unteren Thoraxapertur wird ein Messstrom eingeprägt, ebenso wie zwischen der Strom-Elektrode des oberen Dreifach-Elektrodenelements 80 und des unteren Dreifach-Elektrodenelements 82, wobei der durch die linke Körperhälfte fließende Messstrom und der durch die rechte Körperhälfte fließende Messstrom vorzugweise gleich groß gewählt werden. Durch das Anbringen des linken und des rechten unteren Elektrodenelements 81, 82 wird ein relativ großes Körpervolumen von der Messung erfasst. Alternativ könnte auch nur ein sich über die gesamte Körpervorderseite an der unteren Thoraxapertur erstreckendes Elektroden-Element vorgesehen sein, es hat sich aber gezeigt, dass zwei nebeneinander angeordnete Elektroden-Elemente 81, 82, wie in Fig.9 dargestellt, eine bessere Reproduzierbarkeit der Messergebnisse ermöglichen. Die Messspannungen werden an den zugehörigen ersten und zweiten Spannungs-Elektroden der Elektroden-Elemente 80, 81, 82 abgegriffen und zur Bestimmung von haemodynamischen Parametern in erfindungsgemäßer Weise weiterverarbeitet.

Alle Anschlüsse der Elektroden-Elemente 7, 8a, 80, 81, 82 (und auch der in Fig.10 dargestellten Elektrodenelemente 80', 81', 82', 85, 85', 85") sind über Anschlussleitungen 10 in einem Verteilerstück 9 zusammengeführt, das auf dem Körper des Patienten fixiert ist und einen Winkelmesser 11 beinhaltet, der zur Bestimmung der Lage des Patientenkörpers gegenüber der Horizontalen vorgesehen ist, um den Einfluss derselben auf das Messergebnis festhalten zu können. Der Winkelmesser 11 könnte natürlich auch woanders am Körper des Patienten oder an der Liege, auf der sich das Lebewesen befindet, angebracht sein. Eine Messvorrichtung zur Bestimmung der Impedanz 12 ist über eine mit dem Verteilerstück 9 verbundende Mess- und Steuerleitung 10a in der Lage, alle Beschaltungen der Spannungs-Elektroden und auch der Strom-Elektroden der Elektroden-Elemente 7, 8a, 80, 81, 82, 85 selbsttätig z.B. durch einen Analog-Schalter 13 vorzunehmen.

Die Elektroden-Elemente 80, 81, 82, 81', 82', 85', 85" können neben einer ersten und einer zweiten Spannungs-Elektrode eine dritte Spannungs-Elektrode oder weitere Spannungs-Elektroden aufweisen.

Genauso ist es natürlich denkbar, die bekannten Zirkulärelektroden in 3-oder Mehrfachausführung auf den Körper aufzubringen, bzw. auch für Spotelektroden eine 3-oder Mehrfachausführung anzustreben. Auch jede andere Elektrodenform müsste so ausgeführt sein, dass sich eine veränderbare Distanz zumindest zwischen den Strom- bzw Spannungs-Elektroden ergibt.

Eine weitere beispielsweise Elektrodenanordnung zur Einspeisung des Stroms in der Peripherie ist in Fig. 10 dargestellt. Wie ersichtlich können die beiden Dreifach-Elektrodenelemente 81, 82 an der unteren Thoraxapertur aus der Ausführungsform gemäß Fig.9 durch entsprechende Zweifachelektrodenelemente 81', 82' ersetzt werden, da der Strom über eine Elektrode 7 weiter peripher eingebracht wird, diese ZweifachElektrodenelemente 81', 82' müssen dann allerdings als Doppelspannungselektroden beschaltet sein, um die Impedanz des im wesentlich identen Thoraxsegmentes bei zwei unterschiedlichen Distanzen zu messen.

Zusätzlich zeigt Fig.10 ein eventuell auch zusätzlich anzubringendes Zweifachelektrodenelement 85 am unteren Ende des Rumpfes ca. in Höhe des Schrittes. Alternativ wird in Fig.10a die Platzierung eines Dreifachelektrodenelements 85' am unteren Ende des Rumpfes gezeigt, wo alternativ ebenfalls der Strom eingebracht werden kann und so der Impedanzverlauf des Rumpfes bei zwei verschiedenen Distanzen gemessen werden kann. Dieses Elektrodenelement 85 oder 85' kann entweder als eventuell kurzzuschließendes Doppel- oder Dreifachelektrodenelement links und rechts am Rumpf, oder nur an einer Seite des Rumpfes ausgeführt sein. Diese Elektrodenanordnung hat den Vorteil, daß auch die Impedanz der Extremität bei zwei unterschiedlichen Distanzen vermessen werden kann, sodaß auch hier eine operative Länge bzw. ein elektrisch partizipierendes Volumen errechnet werden kann. Dies ließe sich auch zB. mit einer eventuellen zusätzlichen Staubinde 86 an der Extremität kombinieren, um so in bekannter Weise zusätzlich plethysmographisch die arterielle und venöse Durchblutung zu messen. Für die Messung der venösen Durchblutung müßte nur die Staubinde auf ca. 40 mm Hg, welcher unter dem arteriellen Druck aber höher als der venöse Druck liegt, aufgepumpt werden, um die Volumszunahme des Beines aus der Impedanzänderung zu errechnen. Zur Messung der arteriellen Durchblutung würde man die Staubinde auf Werte höher als der arterielle Blutdruck aufpumpen und dann die Änderung der Impedanz nach Öffnen der Staubinde analysieren. Dies bringt auch eine wesentliche Verbesserung dieser Verfahren, das bisher die Durchblutungsänderung nur als Änderung der Impedanz in Prozent angeben konnte. Mit Hilfe des errechneten elektrisch partizipierenden Volumens kann nun auch Volumsänderung in absolutem Volumen z.B. in Milliliter angegeben werden. Da im wesentlichen beide Beine ein gleiches Volumen aufweisen, könnte auch die doppelseitige Messung an beiden Beinen entfallen und das Elektrodenelement 7 nur an der Peripherie einer Extremität wie in Fig. 10 gezeigt angebracht sein. Dieses einzelne Elektrodenelement könnte dann mit einem einzelnen Elektrodenelement 85 oder 85', oder wie in Fig.10b auch mit einem Doppelspotelektrodenelement 85" kombiniert sein. Sollte die Impedanz der Beine nicht interessieren, könnte auch ein Elektrodenelement 7, das ausschließlich eine einzelne Stromelektrode, aber keine Spannungselektrode beinhaltet, angebracht sein. Auch die Armelektroden könnten, wie in Fig. 10 gezeigt entfallen.

Dies ist deswegen möglich, weil die Arme nur einen kleinen, und sehr konstanten Anteil der elektrisch leitenden Körperflüßigkeiten beinhalten, sodaß auch ohne direkte Vermessung der Arme, aus der Vermessung des Rests des Körpers auf die Gesamtkörperimpedanz und damit auf die Gesamtkörperflüssigkeiten hochgerechnet werden kann. Dies hat den Vorteil, daß trotz einer Überwachung des Patienten mit dem Verfahren und Gerät der Anmeldung die Arme zum Gebrauch durch den Patienten frei bleiben und auch für weitere ärztliche Notwendigkeiten zur Verfügung stehen, was besonders auf Intensivstationen geschätzt wird. Darum wird in der vorliegenden Anmeldung auch wahlweise der Hals, die obere Thoraxapertur, die Arme und auch der Kopf als oberes Körperende gemeint, wenn vom oberen Körperende geschrieben wird.

Auch könnte wahlweise jeweils die linke und/oder die rechten Elektrodenelemente 81, 82, 81', 82' an der unteren Thoraxapertur weggeschaltet werden, um so aus der resultierenden Änderung der Impedanzkurve zusätzlichen Aufschluß über die Richtung der Bewegung des Blutes innerhalb des Thorax noch besser Aufschlüsse über die Funktion des linken und rechten Herzens zu gewinnen. Bekanntlich pumpt das Herz das Blut nach links unten in die links vom Herzen liegende Aorta, sodaß der größte Vektor des Blutes nach links unten geht, dieser Vektor kann mit der wahlweisen Wegschaltung eines der an unteren Thoraxapertur liegenden Elektrodenelemente 81, 82, 81', 82' besser erkannt werden.

Die tatsächliche Anordnung und Ausprägung der Elektrodenelemente als Zweifach- oder Dreifachelektrodenelemente wird sich nach den entsprechenden Anforderungen richten. Es wird versucht werden, mit einem Minimum an Elektrodenelementen das Maximum an Information herauszuholen. Das Minimum an Elektroden ist besonders auf Intensivstationen wichtig, wo eventuell mit einem Zweifachelektrodenelement am Hals 80' und einem oder zwei Dreifachelektrodenelementen am Rumpf 81 das Auslangen gefunden werden muss. Eine weitere sehr ökonomische Variante, jedoch mit einem Maximum an Information ergibt sich aus einem Zweifachelektrodenelement 80' am Hals, zwei weiteren Elektrodenelementen 81', 82' links und rechts an der unteren Thoraxapertur, und ein einzelnes Zweifachelektrodenelement 85 an einer Seite am unteren Ende des Rumofes und eines am entsprechenden unteren Ende derselben unteren Extremität 7. Mit dieser Variante, die nur fünf Zweifachelektrodenelemente enthält, die auch noch für Intensivstationen handhabbar wäre, kann nicht nur extrem genau die Herzleistung, sondern auch zusätzlich die Flüssigkeitsvertielung im Körper aufgegliedert nach Extrazellulärraum und Intrazellulärraum und auch die Flüssigkeitsverschiebung von einer Körperhälfte in die andere erfasst werden. Eine Elnspeisung des Stromes erfolgt dabei einheitlich durch das Elektrodenelement 80' am oberen Ende des Rumpfes und das Elektrodenelement 7 an der unteren Extremität. Die beiden Richtung Körpermitte liegenden Elektroden der Elektrodenelemente 80', 7 sowie die jeweils zwei Elektroden der Elektrodenelemente 81', 82', und des Elementes 85 werden dabei ausschließlich als Spannungselektroden beschaltet.

Außerdem könnte in die Berechnung der Flüssigkeitsvolumina auch noch zusätzlich das Serumnatrium eingehen. Bekanntlich stellt dieses das Hauptjon im Extrazellulärraum dar und macht hiemit hauptsächlich die Leitfähigkeit und der Impedanz aus. Nun kommt es bei Herzkrankheiten, für die das Verfahren und Gerät gemäß der Erfindung ja auch konzipiert ist, oft zu einem deutlichen Absinken des Serumnatriums von einem Normwert von 140 mmol/liter auf bis zu 115 mmol/liter, sodaß die Jonizität und damit die Leitfähigkeit bis um 20 % fallen kann. Dies müßte gegebenenfalls durch eine Eingabe des Serumnatriums oder der Jonizität in die verwendeten Gleichungen berücksichtigt werden. Andererseits beeinflußt ja auch der Hämatokrit die Leitfähigkeit des Blutes, ist dieser annähernd konstant, wie dies in der Regel der Fall ist, kann auch mit den während der Herzaktion gemessenen Änderungen der Impedanz bei mehreren Frequenzen eine Änderung des Serumnatriums erkannt werden.

Eine wichtige Anwendung des Verfahrens kann auch darin liegen, neben der Ausgabe von Schlagvolumen, Auswurffraktion, diastolischer Funktion, Lungen-Wedge-Druck, Gesamtkörperwasser, Extrazellulärraum und Flüssigkeitsverlagerung auch indirekt Hormonkonzentrationen im Blut zu berechnen. Hier seien beispielsweise die Konzentrationen von Natriuretischem Peptid, z.B. Brain-Natriuretic Peptid, oder - Propeptid, atriales natriuretisches Peptid und auch ADH genannt. Die Natriuretischen Peptide werden derzeit im klinischen Alltag zum Screening und zur Diagnose einer Herzinsuffizienz verwendet. Die Bestimmung erfordert eine Blutabnahme und ist sehr teuer (derzeit ungefähr 40 Euro). Bei einer typischen Zahl von ca. 20 Herzpatienten/Tag in einer Ambulanz kann man errechnen, wie rasch sich ein einzelnes Gerät entsprechend der Erfindung amortisiert hätte. Die Regulation dieser Hormone hängt engstens mit dem Flüssigkeitshaushalt und der Dehnung des Herzens durch diese Flüssigkeiten zusammen. Bei detaillierter Kenntnis des Haushaltes der Flüssigkeiten und deren Verschiebung durch die Herzaktion ist es daher möglich, die Konzentration dieser Hormone im Blut vorauszusagen und sich damit die teure Blutbestimmung zu ersparen. Die Ermittlung aller dieser Parameter aus dem erfindungsgemäßen Verfahren erfolgt dabei z.B. empirisch durch Messen der Parameter mit den Goldstandardverfahren, im Fall von den Hormonen durch die Blutbestimmung bei einer repräsentativen Anzahl von Patienten und Schätzung der interessierenden Parameter mit den gemessenen elektrischen Parametern, z.B. mit Hilfe multipler Regressionsgleichungen bzw. auch mit Hilfe neuronaler Netze, anderen "Machine Learning" Algorithmen oder irgend einem anderen Black Box" Modell.

Vom US-Patent US 5 335 667 A von Kichul Cha unterscheidet sich die erfindungsgemäße Elektrodenanordnung ganz wesentlich: In dieser Druckschrift wird zwar der Körper für die Messung der Körperzusammensetzung in Segmente zerlegt, doch kann das im wesentlichen selbe Segment nie mit unterschiedlichen Längen untersucht werden und daher auch keine elektrisch operative Länge errechnet werden. Aus diesem Grunde muß ja auch in der US 5 335 667 A weiterhin als Krücke die Umfangsmessung und die händische Längenmessung des untersuchten Segmentes durchgeführt werden. Dies erfolgt mit dem Zentimetermaß und einem Kaliper wie z.B. in Fig. 5 von US 5 335 667 A gezeigt.

In der gegenständlichen Anmeldung wird jedoch gezeigt, dass die an der Oberfläche gemessene Länge des Segmentes nichts oder kaum etwas mit der errechneten "elektrisch operativen Länge" zu tun hat, wie aus Fig.3 hervorgeht. Und darin liegt ja der ganz wesentliche Vorteil des erfindungsgemäßen Verfahrens. Wie in Fig. 9 und Fig. 10 gezeigt, ist trotz der enormen Aussagekraft des erfindungsgemäßen Verfahrens die Zahl der Elektroden absolut praktikabel, vor allem da sie ja alle als Mehrfachelektroden ausgeführt sind. Die in Abb. 10 gezeigte Elektrodenanordnung hat auch den großen Vorteil, daß jetzt erstmals Lageänderungen entlang der Längsachse des Körpers automatisch erkannt werden können, da jede Lageänderung des Körpers entlang der Längsachse deutliche Flüssigkeitsverschiebungen zur Folge hat. Diese Flüssigkeitsverschiebungen beeinflußen den venösen Rückstrom zum Herzen und damit die Herzleistung, bzw. bewirkt auch eine venöse Insuffizienz bei Aufrichten des Körpers entlang der Längsachse deutlich größere Flüssigkeitsverschiebungen, sodaß auch eine venöse Insuffizienz gut erkannt werden kann. Vor allem in Kombination mit einem Winkelmesser kann dann entschieden werden, ob die Flüssigkeitsverschiebung adäquat oder inadäquat für die vorgebene Lageänderung ist und auch, ob die gemessene Herzleistungsänderung adäquat für die gemessene Flüssigkeitsverschiebung ist.

Diese Untersuchungen lassen sich z.B. hervorragend am Kipptisch ausführen. Auch der elektronische Aufwand ist minimal, weil das Messen bei mehreren Frequenzen und das Umschalten von einer Elektrode auf die andere ja keinerlei teuren oder komplizierten Geräteteile benötigt. Dies macht z.B. auch denkbar, dieses Verfahren auch für die Heimbetreuung des Patienten einzusetzen, indem man ihm ein solches Gerät dem Patienten für zu Hause zur Verfügung stellt. Für diesen Zweck ist die gleichzeitige Miterfassung des EKG natürlich besonders günstig. Die Position der Elektroden am Körper, wie in Fig. 9 und 10 gezeigt, eignet sich auch für die Ableitung des EKG hervorragend. Damit könnte man dem Patienten auch seine eigenen billigen wiederverwendbaren Elektroden zur Verfügung stellen. Diese Elektroden könnten z.B. aus leitfähigem Material mit dehnbaren Bändern gefertigt sein, wie dies von den bei der Sportausübung bekannten Pulsmessgeräten gut erprobt ist. In die z.B. verformbaren Bänder, z.B. aus nicht leitendem Material könnte das leitfähige Material, z.B. bandförmig oder z.B. auch als Spot, z.B. in Form von leitfähigem Gummi, eingearbeitet sein, die dann mittels dehnbarem, öffenbarem, zirkulärem Band am Körper befestigt werden. Diese Elektroden können dann auch leicht vom Patienten, von Angehörigen oder Pflegepersonal angelegt werden.

Diese Resultate müßten dann auch nicht oder nicht komplett im Patientenheimgerät berechnet werden, sondern könnten z.B. per Funk oder über Standleitung, z.B. per Telefon oder Email, einer Zentrale übermittelt werden, wo die Resultate endgültig ausgewertet werden.

Diese unter "Telemedizin" bekannte Entwicklung ist besonders bei Herzpatienten relevant, die derzeit einer engmaschigen Kontrolle in teuren Spezialambulanzen bedürfen. So werden Herzpatienten monatlich und öfters an die teuren Einrichtungen oft mit der Rettung transportiert, um die Therapie zu optimieren. Diese Herzpatienten haben ja oft eine gleich schlechte oder schlechtere Prognose als Krebspatienten und die Therapie muß laufend adaptiert werden, um sie am Leben zu erhalten. Dazu kommt, daß bei der Überalterung der Patienten die Herzkrankheiten und speziell die Herzinsuffizienz enorm zunehmen und bereits jetzt in den Industrieländern zu einer Volksseuche geworden sind. Bei den durch das Verfahren und Gerät der Erfindung erkannten und eventuell per Telemedizin einem Zentrum übermittelten Änderungen, z.B. Verschlechterung von Herzleistung, Überwässerung usw. kann dann entweder der Transport ins Zentrum rechtzeitig erfolgen, oder telefonisch oder per Email die Therapie optimiert werden, bzw. bei guter Funktion des Kreislaufs eine Kontrolle an einem Zentrum eingespart werden. So sind große Geldersparungen trotz des Einsatzes von neuer Technologie gesichert. Bei Übersendung der Rohdaten oder auch nur von grob vorverarbeiteten Daten im Patientengerät kann so lokale Intelligenz in der Messvorrichtung 12 eingespart und so das Gerät noch kostengünstiger gestaltet werden.

Von der Impedanztomographie läßt sich das erfindungsgemäße Verfahren und Gerät insoferne gut abgrenzen, als in der Impedanztomographie jeweils zahlreiche Elektroden auf selber Höhe in der Längsrichtung des Körpers gesehen, angebracht werden, um so aus den in derselben Schnittebene gelegenen Impedanzen eine Abbildung der Flüssigkeitsverteilung in dieser Ebene und aus zahlreichen Ebenen eine dreidimensionale Flüssigkeitsverteilung zu errechnen. Unsere Methode strebt hingegen nicht eine Rekonstruktion einer Ebene, oder die Rekonstruktion der Flüssigkeitsverteilungen im Raum an, sondern die Messung der Flüssigkeitsverschiebung entlang der Längsachse an, wobei erstmals die elektrisch operative Distanz berücksichtigt wird.

Fig. 11 zeigt eine beispielsweise Ausführung einer Mehrfach-Spotelektrode 14, wobei der konstante Elektrodenabstand dabei dadurch gewährleistet werden kann, dass das verbindende Kabel zwischen den Elektroden zum Zeitpunkt der Aufbringung auf den Körper maximal gestreckt wird, und dass durch die Anbringungsart der Elektroden eine Veränderung des Elektrodenabstandes vorzugsweise in Längsachse des Körpers bewerkstelligt wird. Damit der Elektrodenabstand vom Nutzer tatsächlich eingehalten wird, kann auch ein relativ steifer Abstandhalter 16 zwischen den Elektroden vorhanden sein, wobei auch das Verbindungskabel als Elektrodenabstandhalter 16 steif ausgeführt sein kann, wodurch die Einhaltung des Abstandes gewährleistet ist. Bei Berechnung einer operativen Differenz $d_o$ können die Elektroden auch in beliebiger Distanz von einander geklebt werden und ein Distanzhalter erübrigt sich.

[i] DALEN J.E: "The Pulmonary Artery Catheter - Friend, Foe, or Accomplice?" JAMA, July 18, 2001 - Vol 286, No. 3: 348-350.

[ii] POLANCZYK CA, ROHDE LE, GOLDMAN L, COOK EF, THOMAS EJ, MARCANTONIO ER, MANGIONE CM, LEE TH: "Right Heart Cathertization and Cardiac Complications in Patients Undergoing Noncardiac Surgery". JAMA, July 18, 2001 - Vol 286, No. 3: 309-314.

[iii] FORTIN J, NESSLER B, NESSLER W, SKRABAL F: "Medizinische Elektrode", A 392/2001, KL. A61B, eingereicht am 13.03.2001,

[iv] SRAMEK B: "Noninvasive Continuous Cardiac Output Monitor" US 4,450.527, 22. Mai 1984

[v] KUBICEK, W.G., I.N.KARNEGIS, R.P. PATTERSON, D.A. WITSOE, R.H. MATTSON: Development and evaluation of an impedance cardiac output system. Aerospace Medicine 37, 1208 - 1212 (1966)

[vi] KUBICEK, W.G., F.J. KOTTE, M.U. RAMOS, R.P. PATTERSON, D.A. WITSOE, J.W. LA BREE, W. REMOLE, T.E. LAYMAN, H. SCHOENING, D. SMITH: The minnesota impedance cardiograph - theory and applications. Biomed. Eng., 9, 410 - 416, (1974)2

[vii] SRAMEK, B: Noninvasive technique for measurement of cardiac output by means of electrical impedance. Proceedings of the Vth ICEBI Tokyo, (1981)

[viii] SRAMEK, B.BO, D.M. ROSE, A. MIYAMOTO: Stroke volume equation with a linear base impedance model and ist accuracy, as compared to thermodilution and magnetic flowmeter techniques in humans and animals. Proceedings ofthe Vith ICEBI, Zadar, Yugoslavia, S. 38 (1983)

[ix] LAMBERTS, R., K.R. VISSER, W.G. ZIJLSTRA: Impedance cardiography. Van Gorcum, Assen, Holland (1984)

[x] BERNSTEIN, D.P.: A new stroke volume equation for thoracic electrical bioimpedance: Theory and rational. Critical Care Medicin 14, S. 904 - 909 (1986)

[xi] QUAIL, A.W., F.M. TRAUNGOTT, W.L. PORGES: Thoracic resistivity for stroke volume calculation in impedance cardiography J Appl. Physiol.(1981*)*

[xii] SHANKAR, T.M.R., J.G. WEBSTER, S.Y. SHAO: The contribution of vessel volume change and resistivity change to the electrical impedance pulse. IEEE Trans Biomed Engl, BME32:192. (1985*)*

[xiii] WANG L, PATTERSON R: " Multiple Source of the Impedance Cardiogram Based on 3-D Finite Difference Human Thorax Models". IEEE Transactions on Biomedical Engineering Vol.: 42, No:2, 2.February 1995, 141-148

**Patentansprüche**

1. Verfahren zur Messung des Volumens, der Zusammensetzung und Bewegung von elektrisch leitenden Körperflüssigkeiten, beruhend auf der elektrischen Impedanz des Körpers oder eines Körpersegments, insbesondere für die Elektromechanocardiographie (ELMEC)- bzw. Impedanz-Kardiographie (IKG)-Messung zur Bestimmung haemodynamischer Parameter, wobei durch an der Körperoberfläche angelegte Mess-Elektroden ein Wechsel-Messstrom zumindest einer Frequenz in den Körper eingebracht wird, **dadurch gekennzeichnet, dass** die Impedanz und deren Änderung über die Zeit des vom Wechsel-Messstrom durchflossenen, im wesentlichen selben Körpersegmentes bei zumindest zwei unterschiedlichen Messlängen (L, L2, L3, L4, L5) im wesentlichen in Längsrichtung des Körpers gemessen wird.

2. Verfahren nach Anspruch 1, **dadurch gekennzeichnet, dass** die Impedanz am Brustkorb nahe der oberen und unteren. Thoraxapertur bei zumindest zwei verschiedenen Messlängen (L, L2, L3, L4, L5) des im wesentlichen selben Körpersegments abgegriffen wird.

3. Verfahren nach Anspruch 1 und 2, **dadurch gekennzeichnet, dass** der Strom am oder nahe am oberen Körperende, z.B. Hals, Kopf, Arme, und am unteren Körperende, z.B. Bein oder Beine, eingebracht und die Impedanz am Thorax und/oder am Rumpf jeweils bei zumindest zwei verschiedenen Messlängen (L, L2, L3, L4, L5) gemessen wird.

4. Verfahren nach einem der Ansprüche 1 bis 3, **dadurch gekennzeichnet, dass** die Impedanz zumindest einer unteren Extremität bei zumindest zwei verschiedenen Messlängen (L, L2, L3, L4, L5) des im wesentlichen selben Körpersegments abgegriffen wird.

5. Verfahren entweder zur konventionellen Impedanz-Kardiographiemessung oder nach einem der Ansprüche 1 bis 4, **dadurch gekennzeichnet, dass** zusätzlich auch die Gesamtkörper-Impedanz zwischen unterem und oberem Körperende gemessen wird.

6. Verfahren nach Anspruch 1 bis 5, **dadurch gekennzeichnet, dass** die Längendifferenz (d, 1, a, a1) zwischen den beiden verschiedenen Messlängen klein im Verhältnis zur Länge des gemessenen Körperteils ist.

7. Verfahren nach Anspruch 6, **dadurch gekennzeichnet, dass** das Verhältnis der Länge des untersuchten Körperteils zur Längendifferenz (d, d1, a, a1) zwischen 3:1 und 30:1 liegt.

8. Verfahren nach Anspruch 7, **dadurch gekennzeichnet, dass** das Verhältnis der Länge des untersuchten Körperteils zur Längendifferenz (d, d1, a, a1) ungefähr 10:1 beträgt.

9. Verfahren nach Anspruch 1 bis 8, **dadurch gekennzeichnet, dass** aus den bei unterschiedlichen Messlängen (L, L2, L3, L4, L5) zwischen Elektroden ermittelten Impedanzwerten eine operative Elektroden-Messlänge ($L_0$), die der elektrisch operativen Länge des Körpersegments entspricht bzw. gegebenenfalls zusätzlich ein operativer Elektrodenabstand ($d_0$) berechnet wird.

10. Verfahren nach Anspruch 9, **dadurch gekennzeichnet, dass** die elektrisch operative Länge des Körpersegments aus der Formel $L_0 = d/(Z_{02}/Z_{01} - 1)$ errechnet wird, wobei mit d die Längendifferenz zwischen den beiden zur Messung herangezogen Elektroden-Messlängen, mit $Z_{02}$ die Impedanz über die längere Elektroden-Messlänge und mit $Z_{01}$, die Impedanz über die kürzere Elektroden-Messlänge bezeichnet ist.

11. Verfahren nach Anspruch 2, 3, 5 und 6 bis 10, **dadurch gekennzeichnet, dass** die Spannungselektroden an der unteren Thoraxapertur als Doppelelektroden (81, 82, 81', 82') jeweils links und rechts seitlich am Thorax ausgeführt sind, wobei jeweils die in der Längsrichtung auf selber Längendistanz liegenden Elektroden elektrisch miteinander verbunden sind.

**12.** Verfahren nach Anspruch 11, **dadurch gekennzeichnet, dass** wahlweise die jeweils links bzw. rechts an der Thoraxapertur liegenden Elektroden (81, 82, 81', 82') weggeschaltet werden können.

**13.** Verfahren nach Anspruch 3, **dadurch gekennzeichnet, dass** die Spannungselektroden (85, 85', 85") am unteren Ende des Rumpfes als Doppelelektroden jeweils links und rechts am unteren Ende des Rumpfes ausgeführt sind, wobei jeweils die in der Längsrichtung auf selber Längendistanz liegenden Elektroden elektrisch miteinander verbunden sind.

**14.** Verfahren nach Anspruch 13, **dadurch gekennzeichnet, dass** wahlweise die jeweils links bzw. rechts am unteren Ende des Rumpfes liegenden Elektroden (85, 85', 85") weggeschaltet werden können.

**15.** Verfahren nach Anspruch 1 bis 14, **dadurch gekennzeichnet, dass** der Impedanzverlauf nach der Zeit bei zumindest zwei verschiedenen Frequenzen gemessen wird.

**16.** Verfahren nach Anspruch 15, **dadurch gekennzeichnet, dass** die Impedanz bei drei verschiedenen Frequenzen gemessen wird.

**17.** Verfahren nach Anspruch 16, **dadurch gekennzeichnet, dass** die verschiedenen Frequenzen zwischen 1 und 10 kHz, ca. 30 bis 100 kHz und höher als ca.200 kHz betragen.

**18.** Verfahren nach einem der vorhergehenden Ansprüche, **dadurch gekennzeichnet, dass** die maximale zeitliche Änderung der gemessenen Impedanzwerte (dZ/dt) bei zumindest zwei unterschiedlichen Messfrequenzen bestimmt und aus diesen der spezifische Widerstand des im Körper befindlichen Blutes ermittelt wird.

**19.** Verfahren nach Anspruch 18, **dadurch gekennzeichnet, dass** die maximale zeitliche Änderung des gemessenen Impedanzwertes, insbesondere in relativ schmalen Zeitfenstern, zu unterschiedlichen Zeiten der Herzperiode, bestimmt wird.

**20.** Verfahren nach Anspruch 19, **dadurch gekennzeichnet, dass** die Zeitfenster bei einem Steilanstieg des spezifischen Widerstandes und zum Zeitpunkt des minimalen Blutflusses am Ende der Diastole festgesetzt werden.

**21.** Verfahren nach Anspruch 19 und 20, **dadurch gekennzeichnet, dass** die Zeitfenster als schmale Gleitfenster über die gesamte Herzperiode gelegt werden.

**22.** Verfahren nach einem der Ansprüche 15 bis 21, **dadurch gekennzeichnet, dass** die Frequenz des Wechselmessstromes von einer unteren Messfrequenz bis zu einer oberen Messfrequenz kontinuierlich verändert wird.

**23.** Verfahren nach Anspruch 22, **dadurch gekennzeichnet, dass** die untere Messfrequenz ca. 1 kHz und die obere Messfrequenz maximal ca. 1000 kHz beträgt.

**24.** Verfahren nach einem der Ansprüche 15 bis 23, **dadurch gekennzeichnet, dass** der Messstrom bei zumindest einem weiteren Stromelektrodenabstand und bei mehreren unterschiedlichen Messfrequenzen eingeprägt und die Impedanz bei den unterschiedlichen Spannungsmesslängen und unterschiedlichen Frequenzen gemessen wird.

**25.** Verfahren nach einem der Ansprüche 1 bis 24, **dadurch gekennzeichnet, dass** der Phasenwinkel zwischen Messstrom und Messspannung bestimmt wird.

**26.** Verfahren nach einem der Ansprüche 1 bis 25, **dadurch gekennzeichnet, dass** Amplituden, Flächen und Anstiegs- bzw. Abfallstangenten der Impedanz-Wellen B, C, X und O einzeln oder gemeinsam zur Berechnung haemodynamischer Parameter verwendet werden.

**27.** Verfahren nach einem der vorhergehenden Ansprüche, **dadurch gekennzeichnet, dass** empirische Gleichungen, die mit Hilfe eines Goldstandards, wie z.B. des Fick'schen Prinzipes für das Schlagvolumen, bzw. z.B. der Echocardiographie oder Isotopenmethoden für andere Parameter, wie z.B. Auswurfsfraktion, Pulmonalen Wedge-Druck, diastolische Funktion od. dgl. gewonnen wurden, für die Messung haemodynamischer Parameter verwendet werden.

**28.** Verfahren nach einem der Ansprüche 1 bis 27, **dadurch gekennzeichnet, dass** das Serumnatrium bestimmt und zur Berechnung der interessierenden Parameter mitverwendet wird.

**29.** Verfahren nach einem der Ansprüche 1 bis 28, **dadurch gekennzeichnet, dass** die Konzentration des Serumnatriums durch das Verfahren nach einem der Ansprüche 1 bis 27 mathematisch geschätzt und als Resultat ausgegeben wird.

**30.** Verfahren nach einem der Ansprüche 1 bis **29, dadurch gekennzeichnet, dass** die Hormone, wie z.B. ADH und Natriuretisches Peptid, speziell Brain Natriuretic Peptide und deren Vorstufen, die das Körperwasser, dessen Fraktionen und dessen Zusammensetzung regeln, durch das Verfahren nach einem der Ansprüche 1 bis 29 mit Hilfe empirischer Gleichungen geschätzt und als Resultat ausgegeben werden.

**31.** Verfahren nach einem der Ansprüche 1 bis 30, **dadurch gekennzeichnet, dass** die Resultate des Verfahrens digital an eine zentrale Stelle, vorzugsweise per Telefon oder Email, verschickt werden, wo sie weiter verrechnet und beurteilt werden, und daß dem Patienten die nötigen Maßnahmen und Therapieänderungen von einem entfernten Ort aus mitgeteilt werden

**32.** Verfahren nach einem der vorhergehenden Ansprüche, **dadurch gekennzeichnet, dass** das Körperwasser mit seinen Teilfraktionen wie Extrazellulärraum und Intrazellulaärraum ermittelt und ausgegeben wird.

**33.** Gerät zur Messung der elektrischen Impedanz bzw. deren zeitliche Änderung an einem menschlichen Körper, insbesondere für eine Elektromechanocardiographie- bzw. Impedanz-Kardiographie (IKG)-Messung zur Bestimmung haemodynamischer Parameter, zur Durchführung eines Verfahrens nach einem der Ansprüche 1 bis 32, wobei zumindest zwei Spannungselektroden (80, 81, 82, 80' 81', 82') vorgesehen sind, von denen zumindest eine der beiden als Doppelspannungs-Elektrodenelement ausgeführt ist, wobei die Impedanz und deren Änderung nach der Zeit zwischen den zwei Spannungselektroden abgreifbar ist.

**34.** Gerät nach Anspruch 33, **dadurch gekennzeichnet, dass** das zumindest eine Doppelspannungs-Elektrodenelement auf einer gemeinsamen, isolierenden Trägerfolie (2) aufgebracht ist.

**35.** Gerät nach Anspruch 33 und 34, **dadurch gekennzeichnet, dass** zumindest eine der Spannungselektroden (80) aus einem Dreifachelektrodenelement bestehend aus einer Stromelektrode (70) und zwei Spannungselektroden (72, 73) gebildet ist.

**36.** Gerät nach Anspruch 35, **dadurch gekennzeichnet, dass** die Stromelektrode (70) und das Doppelspannungselektrodenelement (72, 73) als Dreifach-Elektrodenelement auf einer gemeinsamen Trägerfolie (2) aufgebracht sind.

**37.** Gerät nach einem der Ansprüche 33 bis 36, **dadurch gekennzeichnet, dass** alle Anschlüsse der Elektroden-Elemente über Anschlußleitungen (10) in einem Verteilerstück (9) zusammengeführt sind, und dass das Verteilerstück (9) mit Messleitungen und Steuerleitungen (10a) einer Messvorrichtung (12) verbunden ist.

**38.** Gerät nach Anspruch 37, **dadurch gekennzeichnet, dass** das Verteilerstück (9) steuerbar ist, sodass die Elektroden-Elemente mit verschiedenen Messleitungen und Steuerleitungen (10a) der Messvorrichtung (12) verbindbar sind.

**39.** Gerät nach einem der Ansprüche 33 bis 38, **dadurch gekennzeichnet, dass** ein Winkelmesser (11) zur Messung der Körperneigung vorgesehen ist.

**40.** Gerät nach Anspruch 39, **dadurch gekennzeichnet, dass** der Winkelmesser (11) auf dem Verteilerstück (9) angeordnet ist.

**41.** Medizinisches Elektroden-Element zur Messung der elektrischen Impedanz bzw. deren zeitlichen Änderung an einem menschlichen Körper, insbesondere für eine Elektromechanocardiographie oder Impedanz-Kardiographie (IKG)-Messung zur Bestimmung haemodynamischer Parameter, mit einer ersten Strom-Elektrode (70), die einen Strom-Anschluss (90) zum Einprägen eines elektrischen Wechsel-Messstromes aufweist, und einer von dieser beabstandeten, ersten Spannungs-Elektrode (73, 15), die einen Spannungs-Anschluss (93) zum Abgreifen einer elektrischen Messspannung aufweist, wobei zumindest eine weitere Spannungs-Elektrode (71, 72) mit einem Spannungs-Anschluss vorgesehen ist, und wobei die zumindest eine weitere Spannungs-Elektrode (71, 72) in einem Abstand (d, d1) zur ersten Spannungs-Elektrode (73, 15) angeordnet ist bzw. sind zur Durchführung des Verfahrens nach einem der Ansprüche 1 bis 32 wobei die erste Spannungs-Elektrode (73) sowie die zumindest eine weitere Spannungs-Elektrode (71, 72) in Form von zueinander parallelen, elektrisch leitenden Streifen ausgebildet sind, wobei die Streifenbreite gleich oder bevorzugt kleiner als der Abstand zwischen den Streifen ist.

42. Medizinisches Elektroden-Element nach Anspruch 41, **dadurch gekennzeichnet, dass** das Verhältnis der Streifenlänge zum Elektrodenabstand (a, a1, d, d1) im Bereich zwischen 2 und 15 liegt.

43. Medizinisches Elektroden-Element nach Anspruch 41 und 42, **dadurch gekennzeichnet, dass** die erste Spannungs-Elektrode (73) und die erste Strom-Elektrode (70) sowie die zumindest eine weitere Spannungs-Elektrode (71, 72) und/oder zumindest eine weitere Strom-Elektrode in Form von zueinander parallelen, elektrisch leitenden Streifen ausgebildet sind, wobei die Streifenbreite gleich oder bevorzugt kleiner als der Abstand zwischen den Streifen ist.

44. Medizinisches Elektroden-Element nach einem der Ansprüche 41 bis 43, **dadurch gekennzeichnet, dass** die erste Spannungs-Elektrode (73) und die erste Strom-Elektrode (70) sowie die zumindest eine weitere Spannungs-Elektrode (71, 72) auf einem gemeinsamen elektrisch isolierenden Trägermaterial (2, 3, 4) angeordnet sind.

45. Medizinisches Elektroden-Element nach Anspruch 41 bis 44, **dadurch gekennzeichnet, dass** das Trägermaterial durch eine Trägerfolie (2) gebildet ist, und dass die erste Spannungs-Elektrode (73) und die erste Strom-Elektrode (70) sowie die zumindest eine weitere Spannungs-Elektrode (72) und/oder die zumindest eine weitere Strom-Elektrode auf einer Seite der Trägerfolie (2) auf diese aufgebracht und vorzugsweise mit einer elektrisch leitenden Klebstoffschicht versehen sind.

46. Medizinisches Elektroden-Element nach einem der Ansprüche 41 bis 44, **dadurch gekennzeichnet, dass** das Trägermaterial mehrere Folienstreifen (3) mit einer klebfähigen Oberfläche umfasst, auf welche die erste Spannungs-Elektrode (73) und die erste Strom-Elektrode (70) sowie die zumindest eine weitere Spannungs-Elektrode (72) aufgebracht sind, und dass die Folienstreifen (3) mit den darauf befindlichen Elektroden (70, 72, 73) in im Wesentlichen paralleler Anordnung auf einer gemeinsamen Basis-Trägerfolie (4) haften, welche Basis-Trägerfolie (4) nach Aufbringen der Folienstreifen (3) auf die Körperoberfläche von diesen abziehbar ist.

47. Medizinisches Elektroden-Element nach einem der Ansprüche 41 bis 46, **dadurch gekennzeichnet, dass** die Trägerfolie (2, 4) an einem Längsende sich auf einer Seite zu einer Steck-Anschlussfläche (6) verjüngt, auf der die erste Spannungs-Elektrode (73) und die erste Strom-Elektrode (70) sowie die zumindest eine weitere Spannungs-Elektrode (72) und/oder die zumindest eine weitere Strom-Elektrode eng beabstandet geführt sind.

48. Medizinisches Elektroden-Element nach einem der Ansprüche 33 bis 40, **dadurch gekennzeichnet, dass** die erste Spannungs-Elektrode und die erste Strom-Elektrode sowie die zumindest eine weitere Spannungs-Elektrode und/oder die zumindest eine weitere Strom-Elektrode in Form von Spot-Elektroden (14) ausgeführt sind, die durch Abstandhalter (16, 17), z.B. auch durch eine gemeinsame Trägerfolie (85") voneinander beabstandet angeordnet sind.

49. Medizinisches Elektroden-Element nach einem der Ansprüche 33 bis 40, sowie nach den Ansprüchen 41 bsi 43 sowie nach Anspruch 48, **dadurch gekennzeichnet, dass** die Elektrodenelemente aus wiederverwendbaren, zumindest teilweise biegsamen und/oder elastischen, elektrisch nicht oder schlecht leitenden, zirkulären, zu öffnenden Bändern bestehen, in die das leitfähige Material der Elektroden z.B. bandförmig oder spotförmig eingearbeitet ist.

50. Gerät nach einem der Ansprüche 33 bis 49, **dadurch gekennzeichnet, dass** die Elektroden oder Elektrodenelemente auch als EKG-Elektroden beschaltet sind.

## Claims

1. A method for measuring the volume, the composition and the movement of electroconductive body fluids, based on the electrical impedance of the body or a body segment, in particular for performing electromechanocardiography (ELMEC) or impedance cardiography (IKG) measurements for determining hemodynamic parameters, wherein an alternating measuring current of at least one frequency is introduced into the body by means of measuring electrodes attached to the body surface, **characterized in that** the impedance and temporal variations thereof of essentially the same body segment through which the alternating measuring current flows are measured for at least two different measuring lengths (L, L2, L3, L4, L5), essentially in the longitudinal direction of the body.

**2.** The method according to claim 1, **characterized in that** the impedance is sensed at the thorax close to the inferior and superior thoracic apertures for at least two different measuring lengths (L, L2, L3, L4, L5) of essentially the same body segment.

**3.** The method according to the claims 1 and 2, **characterized in that** the electrical current is introduced at or close to the upper body end, such as at the neck, the head, the arms, and the lower body end, such as at a leg or at both legs, and that the impedance is measured at the thorax and/or trunk for at least two different measuring lengths (L, L2, L3, L4, L5).

**4.** The method according to any one of claims 1 to 3, **characterized in that** the impedance of at least one lower extremity is sensed for at least two different measuring lengths (L, L2, L3, L4, L5) of essentially the same body segment.

**5.** A method for either performing conventional impedance cardiography measurements or according to any one of claims 1 to 4, **characterized in that** additionally the whole-body impedance between the lower und upper body ends is measured.

**6.** The method according to any one of claims 1 to 5, **characterized in that** the length difference (d, 1, a, a1) between the two different measuring lengths is small compared to the length of the examined body part.

**7.** The method according to claim 6, **characterized in that** the ratio of the length of the examined body part to the length difference (d, d1, a, a1) lies between 3:1 and 30:1.

**8.** The method according to claim 7, **characterized in that** the ratio of the length of the examined body part to the length difference (d, d1, a, a1) is approximately 10:1.

**9.** The method according to any one of claims 1 to 8, **characterized in that** on the basis of the impedance values determined for the different measuring lengths (L, L2, L3, L4, L5) between electrodes an operational electrode measuring length ($L_0$), which corresponds to the electrically operational length of the body segment, and optionally an operational electrode distance ($d_0$) are calculated.

**10.** The method according to claim 9, **characterized in that** the electrically operational length of the body segment is calculated according to the formula $L_0 = d/(Z_{02}/Z_{01} - 1)$, wherein d is the difference between the two electrode measuring lengths used for the measurement, $Z_{02}$ is the impedance for the longer electrode measuring length, and $Z_{01}$ is the impedance for the shorter electrode measuring length.

**11.** The method according to any one of claims 2, 3, 5, and 6 to 10, **characterized in that** the voltage electrodes at the inferior thoracic aperture are provided as double electrodes (81, 82, 81', 82') on the left and right side, respectively, of the thorax, wherein the electrodes positioned at the same distance in the longitudinal direction are electrically connected to each other.

**12.** The method according to claim 11, **characterized in that** the electrodes (81, 82, 81', 82') on the left and right side, respectively, of the thoracic aperture may selectively be switched off.

**13.** The method according to claim 3, **characterized in that** the voltage electrodes (85, 85', 85'') at the lower end of the trunk are provided as double electrodes on the left and right side, respectively, of the lower end of the trunk, wherein the electrodes positioned at the same distance in the longitudinal direction are electrically connected to each other.

**14.** The method according to claim 13, **characterized in that** the electrodes on the left and right side, respectively, of the lower end of the trunk may selectively be switched off.

**15.** The method according to any one of claims 1 to 14, **characterized in that** the impedance variations with respect to time are measured at at least two frequencies.

**16.** The method according to claim 15, **characterized in that** the impedance is measured at three different frequencies.

**17.** The method according to claim 16, **characterized in that** the different frequencies amount to between 1 and 10 kHz, approximately 30 to 100 kHz and above approximately 200 kHz.

18. The method according to any one of the preceding claims, **characterized in that** the maximum temporal variation of the measured impedance values (dZ/dt) is determined at at least two different measuring frequencies, and that the resistivity of the blood present in the body is determined therefrom.

19. The method according to claim 18, **characterized in that** the maximum temporal variation of the measured impedance value, especially in relatively narrow time frames, is determined at different times of the cardiac cycle.

20. The method according to claim 19, **characterized in that** the time frames are set at an abrupt rise of resistivity and at the time of minimum blood flow at the end of a diastole.

21. The method according to the claims 19 and 20, **characterized in that** the time frames are cover the whole cardiac cycle in the form of narrow gliding frames.

22. The method according to any one of claims 15 to 21, **characterized in that** the frequency of the alternating measuring current is continuously varied from a lower measuring frequency to a higher measuring frequency.

23. The method according to claim 22, **characterized in that** the lower measuring frequency amounts to approximately 1 kHz and the higher measuring frequency to a maximum of approximately 1000 kHz.

24. The method according to any one of claims 15 to 23, **characterized in that** the measuring current is impressed for at least one other current electrode distance and at several different measuring frequencies, and that the impedance is measured for the different voltage measuring lengths and at the different frequencies.

25. The method according to any one of claims 1 to 24, **characterized in that** the phase angle between the measuring current and the measuring voltage is determined.

26. The method according to any one of claims 1 to 25, **characterized in that** amplitudes, areas and ascending or descending tangents of the impedance waves B, C, X and O are used independently or together for calculating hemodynamic parameters.

27. The method according to any one of the preceding claims, **characterized in that** empirical equations that have been determined by means of a gold standard, for example by means of the Fick principle for stroke volume, or, for example, echocardiography or isotope methods for other parameters, such as ejection fraction, pulmonary wedge pressure, diastolic function and the like, are used for measuring hemodynamic parameters.

28. The method according to any one of claims 1 to 27, **characterized in that** the sodium content in serum is determined and used in the calculation of relevant parameters.

29. The method according to any one of claims 1 to 28, **characterized in that** the sodium concentration in serum is mathematically estimated by the method according to any one of claims 1 to 27 and obtained as a result thereof.

30. The method according to any one of claims 1 to 29, **characterized in that** the hormones, such as ADH and natriuretic peptide, especially brain natriuretic peptides and precursors thereof, regulating the body water as well as its fractions and composition, are estimated by the method according to any one of claims 1 to 29 by means of empirical equations and obtained as a result thereof.

31. The method according to any one of claims 1 to 30, **characterized in that** the results obtained by means of said method are sent, preferably by telephone or e-mail, in digital form to a central station, where they are further processed and assessed, and that all necessary measures and therapy changes are transmitted to the patient from a remote place.

32. The method according to any one of the preceding claims, **characterized in that** the body water is determined with its fractions, such as extracellular space and intracellular space, and read out.

33. An apparatus for measuring the electrical impedance or temporal variations thereof in a human body, in particular for performing electromechanocardiography or impedance cardiography (IKG) measurements for determining hemodynamic parameters, for implementing a method according to any one of claims 1 to 32, wherein at least two voltage electrodes (80, 81, 82, 80', 81', 82') are provided, at least one of which is provided as double voltage electrode element, wherein the impedance and the temporal variations thereof may be sensed between said two voltage electrodes.

34. The apparatus according to claim 33, **characterized in that** said at least one double voltage electrode element is attached to a common insulating carrier sheet (2).

35. The apparatus according to the claims 33 and 34, **characterized in that** at least one of the voltage electrodes (80) is formed of a triple electrode element consisting of a current electrode (70) and two voltage electrodes (72, 73).

36. The apparatus according to claim 35, **characterized in that** the current electrode (70) and the double voltage electrode element (72, 73) are attached to a common carrier sheet (2) in the form of a triple electrode element.

37. The apparatus according to any one of claims 33 to 36, **characterized in that** all the terminals of the electrode elements are brought together in a distribution element (9) by means of connecting leads (10), and that the distribution element is connected to measuring lines and control lines (10a) of a measuring device (12).

38. The apparatus according to claim 37, **characterized in that** the distribution element (9) can be controlled so that the electrode elements can be connected to different measuring lines and control lines (10a) of the measuring device (12).

39. The apparatus according to any one of claims 33 to 38, **characterized in that** an angle meter (11) for measuring body inclination is provided.

40. The apparatus according to claim 39, **characterized in that** the angle meter (11) is positioned on the distribution element (9).

41. A medical electrode element for measuring the electrical impedance or temporal variations thereof in a human body, in particular for performing electromechanocardiography or impedance cardiography (IGK) measurements for determining hemodynamic parameters, by means of a first current electrode (70), which has an electrical terminal for impressing an alternating measuring current, and a first voltage electrode (73, 15), which has a voltage terminal for sensing an electrical measuring voltage, wherein at least one further voltage electrode (71, 72) with a voltage terminal is provided, and wherein the at least one further voltage electrode (71, 72) is positioned at a distance (d, d1) from the first voltage electrode (73, 15), for implementing the method according to any one of claims 1 to 32, wherein the first voltage electrode (73) as well as the at least one further voltage electrode (71, 72) are provided as parallel, electroconductive strips, the widths of the strips being equal to or preferably smaller than the distance between the strips.

42. The medical electrode element according to claim 41, **characterized in that** the ratio of the length of the strips to the distance between the electrodes (a, a1, d, d1) lies in the range between 2 and 15.

43. The medical electrode element according to the claims 41 and 42, **characterized in that** the first voltage electrode (73) and the first current electrode (70) as well as the at least one further voltage electrode (71, 72) and/or at least one further current electrode are provided as parallel, electroconductive strips, the widths of the strips being equal to or preferably smaller than the distance between the strips.

44. The medical electrode element according to any one of claims 41 to 43, **characterized in that** the first voltage electrode (73) and the first current electrode (70) as well as the at least one further voltage electrode (71, 72) are positioned on a common, electrically insulating carrier material (2, 3, 4).

45. The medical electrode element according to any one of claims 41 to 44, **characterized in that** the carrier material consists of a carrier sheet (2), and that the first voltage electrode (73) and the first current electrode (70) as well as the at least one further voltage electrode (72) and/or the at least one further current electrode are attached to one side of the carrier sheet and preferably provided with an electroconductive adhesive layer.

46. The medical electrode element according to any one of claims 41 to 44, **characterized in that** the carrier material comprises a plurality of sheet strips (3) with adhesive surfaces, to which the first voltage electrode (73) and the first current electrode (70) as well as the at least one further voltage electrode (72) are attached, and that the sheet strips (3) with the electrodes (70, 72, 73) secured thereon adhere to a common base carrier sheet (4) in a substantially parallel arrangement, wherein said base carrier sheet (4) may be peeled off from the sheet strips (3) after they have been attached to the body surface.

**47.** The medical electrode element according to any one of claims 41 to 46, **characterized in that** the carrier sheet (2, 4) at one longitudinal end is tapered to form a plug-type surface (6), on which the first voltage electrode (73) and the first current electrode (70) as well as the at least one further voltage electrode (72) and/or the at least one further current electrode are provided in a closely spaced way.

**48.** The medical electrode element according to any one of claims 33 to 40, **characterized in that** the first voltage electrode and the first current electrode as well as the at least one further voltage electrode and/or the at least on further current electrode are provided in the form of spot electrodes (14), which are spaced from each other by means of spacers (16, 17), such as by a common carrier sheet (85").

**49.** The medical electrode element according to any one of claims 33 to 40 as well as any one of the claims 41 to 43 as well as claim 48, **characterized in that** the electrode elements consist of reusable, at least partially flexible and/or elastic, electrically nonconductive or poorly conductive, circular, openable bands, into which the conductive material of the electrodes is incorporated, e.g. in the form of strips or spots.

**50.** The apparatus according to any one of claims 33 to 49, **characterised in that** the electrodes or electrode elements are also operated as ECG electrodes.

**Revendications**

**1.** Méthode pour mesurer le volume, la composition et le mouvement de liquides organiques électroconducteurs, basée sur l'impédance électrique du corps ou d'un segment du corps, spécialement pour la mesure d'une électro-mécano-cardiographie (ELMEC) ou d'une cardiographie de l'impédance (CGI) pour déterminer des paramètres hémodynamiques, un courant alternatif de mesure à au moins une fréquence étant introduit dans le corps via des électrodes de mesure positionnées sur la surface du corps, **caractérisée en ce que** l'impédance et les variations temporelles de celle-ci du sensiblement même segment du corps, dans lequel circule ledit courant alternatif de mesure, sont mesurées pour au moins deux longueurs de mesure différentes (L, L2, L3, L4, L5), sensiblement dans la direction longitudinale du corps.

**2.** Méthode selon la revendication 1, **caractérisée en ce que** l'impédance est prélevée au thorax près de l'ouverture inférieure et supérieure de ceci pour au moins deux longueurs de mesure différentes (L, L2, L3, L4, L5) du sensiblement même segment du corps.

**3.** Méthode selon les revendications 1 et 2, **caractérisée en ce que** le courant est introduit à l'extrémité supérieure du corps ou près de celle-ci, p.ex. au cou, à la tête, aux bras, et à l'extrémité inférieure du corps, p.ex. à une ou aux deux jambes, et **en ce que** l'impédance au thorax et/ou au tronc est mesurée respectivement pour deux longueurs de mesure différentes (L, L2, L3, L4, L5).

**4.** Méthode selon l'une des revendications 1 à 3, **caractérisée en ce que** l'impédance d'au moins une extrémité inférieure est prélevée pour au moins deux longueurs de mesure différentes (L, L2, L3, L4, L5) du sensiblement même segment du corps.

**5.** Méthode pour la mesure conventionnelle de la cardiographie de l'impédance ou selon l'une des revendications 1 à 4, **caractérisée en ce que**, en plus, l'impédance du corps entier est mesurée entre l'extrémité inférieure et l'extrémité supérieure du corps.

**6.** Méthode selon l'une des revendications 1 à 5, **caractérisée en ce que** la différence de longueur (d, 1, a, a1) entre les deux longueurs de mesure différentes est petite par rapport à la longueur du segment du corps examiné.

**7.** Méthode selon la revendication 6, **caractérisée en ce que** le rapport entre la longueur du segment du corps examiné et la différence de longueur (d, d1, a, a1) est compris entre 3:1 et 30:1.

**8.** Méthode selon la revendication 7, **caractérisée en ce que** le rapport entre la longueur du segment du corps examiné et la différence de longueur (d, d1, a, a1) s'élève à environ 10:1.

**9.** Méthode selon l'une des revendications 1 à 8, **caractérisée en ce qu'**une longueur de mesure d'électrodes opérationnelle ($L_0$), qui correspond à la longueur électriquement opérationnelle du segment du corps, et facultativement une distance opérationnelle d'électrodes ($d_0$) sont calculées à partir des valeurs d'impédance déterminées par rapport aux longueurs de mesure différentes (L, L2, L3, L4, L5) entre les électrodes.

**10.** Méthode selon la revendication 9, **caractérisée en ce que** la longueur électriquement opérationnelle du segment du corps est calculée à l'aide de la formule $L_0 = d/(Z_{02}/Z_{01} - 1)$, d représentant la différence de longueur entre les deux longueurs de mesure d'électrodes utilisées pour la mesure, $Z_{02}$ représentant l'impédance de la longueur de mesure d'électrodes plus longue et $Z_{01}$ représentant l'impédance de la longueur de mesure d'électrodes plus courte.

**11.** Méthode selon l'une des revendications 2, 3, 5, et 6 à 10, **caractérisée en ce que** les électrodes de tension à l'ouverture inférieure du thorax sont disposées en forme d'électrodes doubles (81, 82, 81', 82') respectivement sur le côté gauche et droite du thorax, les électrodes situées à la même distance dans la direction longitudinale étant connectées électriquement l'une avec l'autre.

**12.** Méthode selon la revendication 11, **caractérisée en ce que** les électrodes positionnées sur le côté gauche ou droite de l'ouverture du thorax (81, 82, 81', 82') peuvent être déconnectées sélectivement.

**13.** Méthode selon la revendication 3, **caractérisée en ce que** les électrodes de tension (85, 85', 85") à l'extrémité inférieure du tronc sont disposées en forme d'électrodes doubles respectivement sur le côté gauche ou droite de ladite extrémité inférieure du tronc, les électrodes situées à la même distance dans la direction longitudinale étant connectées électriquement l'une avec l'autre.

**14.** Méthode selon la revendication 13, **caractérisée en ce que** les électrodes situées sur le côté gauche ou droite de l'extrémité inférieure du tronc (85, 85', 85") peuvent être déconnectées sélectivement.

**15.** Méthode selon l'une des revendications 1 à 14, **caractérisée en ce que** la variation temporelle de l'impédance est mesurée à au moins deux fréquences différentes.

**16.** Méthode selon la revendication 15, **caractérisée en ce que** l'impédance est mesurée à trois fréquences différentes.

**17.** Méthode selon la revendication 16, **caractérisée en ce que** les fréquences différentes s'élèvent à 1 et 10 kHz, à environ 30 à 100 kHz et à plus d'environ 200 kHz.

**18.** Méthode selon l'une des revendications précédentes, **caractérisée en ce que** la variation temporelle maximale des valeurs d'impédance mesurées (dZ/dt) est déterminée à au moins deux fréquences de mesure différentes et **en ce que** la résistivité du sang dans le corps est déterminée à partir desdites valeurs d'impédance.

**19.** Méthode selon la revendication 18, **caractérisée en ce que** la variation temporelle maximale de la valeur d'impédance mesurée, spécialement pendant des intervalles relativement courts, est déterminée à des points différents du cycle cardiaque.

**20.** Méthode selon la revendication 19, **caractérisée en ce que** lesdits intervalles sont fixés à une montée abrupte de la résistivité et au moment de la circulation sanguine minimale à la fin de la diastole.

**21.** Méthode selon les revendications 19 et 20, **caractérisée en ce que** les intervalles couvrent le cycle cardiaque entier en forme d'intervalles glissants.

**22.** Méthode selon l'une des revendications 15 à 21, **caractérisée en ce que** la fréquence du courant alternatif de mesure est variée d'une manière continue à partir d'une fréquence de mesure inférieure jusqu'à une fréquence de mesure supérieure.

**23.** Méthode selon la revendication 22, **caractérisée en ce que** la fréquence de mesure inférieure s'élève à environ 1 kHz et la fréquence de mesure supérieure à environ 1000 kHz au maximum.

**24.** Méthode selon l'une des revendications 15 à 23, **caractérisée en ce que** le courant de mesure est appliqué avec au moins une distance additionnelle des électrodes de courant et à plusieurs fréquences de mesure différentes et **en ce que** l'impédance est mesurée à des longueurs de mesure de tension différentes et à des fréquences différentes.

**25.** Méthode selon l'une des revendications 1 à 24, **caractérisée en ce que** l'angle de phase entre le courant de mesure et la tension de mesure est déterminé.

**26.** Méthode selon l'une des revendications 1 à 25, **caractérisée en ce que** les amplitudes, les surfaces et les tangentes montantes et descendantes des ondes d'impédance B, C, X et O sont, individuellement ou en combinaison, utilisées pour calculer des paramètres hémodynamiques.

**27.** Méthode selon l'une des revendications précédentes, **caractérisée en ce que** des équations empiriques, qui ont été déterminées à l'aide d'un standard d'or, p.ex. à l'aide du principe de Fick pour le débit systolique ou, p.ex., de méthodes échocardographiques ou isotopiques pour autres paramètres, p.ex. la fraction d'éjection, la pression pulmonaire Wedge, la fonction diastolique etc., sont utilisées pour la mesure de paramètres hémodynamiques.

**28.** Méthode selon l'une des revendications 1 à 27, **caractérisée en ce que** la teneur en sodium dans le sérum est déterminée et utilisée pour calculer les paramètres d'intérêt.

**29.** Méthode selon l'une des revendications 1 à 28, **caractérisée en ce que** la concentration du sodium dans le sérum est estimée mathématiquement par la méthode selon l'une des revendications 1 à 27 et obtenue comme résultat de celle-ci.

**30.** Méthode selon l'une des revendications 1 à 29, **caractérisée en ce que** les hormones, p.ex. l'AVP et le peptide natriurétique, spécialement les peptides cérébraux natriurétiques et leurs précurseurs, qui règlent le liquide organique, ses fractions et sa composition, sont estimées par la méthode selon l'une des revendications 1 à 29 à l'aide d'équations empiriques et obtenues comme résultat de celle-ci.

**31.** Méthode selon l'une des revendications 1 à 30, **caractérisée en ce que** les résultats de la méthode sont envoyés, en forme numérique, de préférence par téléphone ou e-mail, à un lieu central, où ils sont traités et évalués, et **en ce que** les mesures et les changements de thérapie nécessaires sont communiqués au patient à partir d'un lieu éloigné.

**32.** Méthode selon l'une des revendications précédentes, **caractérisée en ce que** le liquide organique est déterminé avec ses fractions, p.ex. l'espace extracellulaire et intracellulaire, et sorti.

**33.** Appareil pour mesurer l'impédance électrique ou les variations temporelles de celle-ci dans un corps humain, spécialement pour la mesure d'une électro-mécano-cardiographie (ELMEC) ou d'une cardiographie de l'impédance (CGI) pour déterminer des paramètres hémodynamiques, pour exécuter la méthode selon l'une des revendications 1 à 32, dans laquelle au moins deux électrodes de tension (80, 81, 82, 80', 81', 82') sont prévues, dont au moins une étant disposée en forme d'un élément d'électrode de tension double, l'impédance et ses variations temporelles étant prélevées entre les deux électrodes de tension.

**34.** Appareil selon la revendication 33, **caractérisé en ce que** ledit au moins un élément d'électrode de tension double est fixé sur une feuille (2) isolante commune.

**35.** Appareil selon les revendications 33 et 34, **caractérisé en ce que** au moins une des électrodes de tension (80) est formée par un élément d'électrode triple étant composé d'une électrode de courant (70) et de deux électrodes de tension (72, 73).

**36.** Appareil selon la revendication 35, **caractérisé en ce que** l'électrode de courant (70) et l'élément d'électrode de tension double (72, 73) sont fixés sur une feuille support (2) commune en forme d'un élément d'électrode triple.

**37.** Appareil selon l'une des revendications 33 à 36, **caractérisé en ce que** tous les raccords des éléments d'électrode sont réunis dans un élément distributeur (9) via des lignes de raccordement (10) et **en ce que** l'élément distributeur (9) est connecté avec les lignes de mesure et les lignes pilotes (10a) d'un dispositif de mesure (12).

**38.** Appareil selon la revendication 37, **caractérisé en ce que** l'élément distributeur (9) peut être commandé ainsi que les éléments d'électrode peuvent être connectés avec des lignes de mesure et des lignes pilotes (10a) différentes dudit dispositif de mesure (12).

39. Appareil selon l'une des revendications 33 à 38, **caractérisé en ce qu'**un dispositif de mesure d'angle (11) pour mesurer l'inclinaison du corps est prévu.

40. Appareil selon la revendication 39, **caractérisé en ce que** ledit dispositif de mesure d'angle (11) est disposé sur ledit élément distributeur (9).

41. Élément d'électrode médical pour mesurer l'impédance électrique du corps ou d'un segment du corps, spécialement pour la mesure d'une électro-mécano-cardiographie (ELMEC) ou d'une cardiographie de l'impédance (CGI) pour déterminer des paramètres hémodynamiques, avec une première électrode de courant (70) ayant un raccord électrique (90) pour l'application d'un courant alternatif de mesure et avec une première électrode de tension (73, 15) écartée de celle-ci ayant un raccord de tension (93) pour le prélèvement d'une tension électrique de mesure, au moins une autre électrode de tension (71, 72) avec un raccord de tension étant prévue et ladite au moins une autre électrode de tension (71, 72) étant disposée à une distance (d, d1) de la première électrode de tension (73, 15), pour exécuter la méthode selon l'une des revendications 1 à 32, la première électrode de tension (73) et ladite au moins une autre électrode de tension (17, 72) étant formées en forme de bandes électroconductrices parallèles, la largeur des bandes étant égale à ou, de préférence, plus petite que la distance entre les bandes.

42. Élément d'électrode médical selon la revendication 41, **caractérisé en ce que** le rapport entre la longueur des bandes et la distance entre les électrodes (a, a1, d, d1) est compris entre 2 et 15.

43. Élément d'électrode médical selon les revendications 41 et 42, **caractérisé en ce que** la première électrode de tension (73) et la première électrode de courant (70) ainsi que ladite au moins une autre électrode de tension (71, 82) et/ou ladite au moins une autre électrode de courant sont formées en forme de bandes électroconductrices parallèles, la largeur des bandes étant égale à ou, de préférence, plus petite que la distance entre les bandes.

44. Élément d'électrode médical selon l'une des revendications 41 à 43, **caractérisé en ce que** la première électrode de tension (73) et la première électrode de courant (70) ainsi que ladite au moins une autre électrode de tension (71, 72) sont disposées sur un matériau support isolant (2, 3, 4).

45. Élément d'électrode médical selon l'une des revendications 41 à 44, **caractérisé en ce que** le matériau support consiste en une feuille support (2) et **en ce que** la première électrode de tension (73) et la première électrode de courant (70) ainsi que ladite au moins une autre électrode de tension (72) et /ou ladite au moins une autre électrode de courant sont fixées sur une côté de la feuille support (2) et, de préférence, munies d'une couche adhésive électroconductrice.

46. Élément d'électrode médical selon l'une des revendications 41 à 44, **caractérisé en ce que** le matériau support consiste en plusieurs bandes de feuille (3) avec une surface adhésive, sur laquelle la première électrode de tension (73) et la première électrode de courant (70) ainsi que ladite au moins une autre électrode de tension sont fixées, et **en ce que** les bandes de feuille (3) et les électrodes (70, 72, 73) fixées sur celles-ci sont attachées sur une feuille support de base commune (4) dans une disposition sensiblement parallèle, ladite feuille support de base (4) pouvant être pelée des bandes de feuille (3) après la fixation de celles-ci sur la surface du corps.

47. Élément d'électrode médical selon l'une des revendications 41 à 46, **caractérisé en ce que** la feuille support (2, 4) s'effile à une extrémité longitudinale pour former une face de prise de courant embrochable, sur laquelle la première électrode de tension (73) et la première électrode de courant (70) ainsi que ladite au moins une autre électrode de tension (72) et/ou ladite au moins une autre électrode de courant sont disposées à courte distance l'une de l'autre.

48. Élément d'électrode médical selon l'une des revendications 33 à 40, **caractérisé en ce que** la première électrode de tension et la première électrode de courant ainsi que ladite au moins une autre électrode de tension et/ou ladite au moins une électrode de courant sont prévues en forme d'électrodes ponctuelles (14) qui sont écartées l'une de l'autre à l'aide d'écarteurs (16, 17), p.ex. à l'aide d'une feuille support commune (85").

49. Élément d'électrode médical selon l'une des revendications 33 à 40 ainsi que selon l'une des revendications 41 à 43 ainsi que selon la revendication 48, **caractérisé en ce que** les éléments d'électrode se composent de bandes réutilisables, au moins partiellement flexibles et/ou élastiques, non conductrices ou mauvaises conductrices, circulaires et ouvrables dans lesquelles le matériau conducteur des électrodes est incorporé en forme de points ou de bandes.

**50.** Appareil selon l'une des revendications 33 à 49, **caractérisé en ce que** les électrodes ou les éléments d'électrode sont actionnés aussi en qualité d'électrodes ECG.

FIG.1

FIG.2

FIG.3

FIG.4A

FIG.4B

FIG.5

FIG.6

FIG.7

FIG.8

Fig. 9

9

11

10

80'

81'

82'

10a

85'

85

86

7

Fig. 10a

Fig. 10b

85"

Fig. 10

16

14

15

Fig. 11

L2 = L + d
L3 = L + d1

**FIG.12**

L4 = L + a
L5 = L + a1

**FIG.13**

IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4450527 A
- US 5109870 A
- US 4951682 A
- US 4947862 A
- US 5063937 A
- US 5335667 A


**In der Beschreibung aufgeführte Nicht-Patentliteratur**

- **DALEN J.E.** The Pulmonary Artery Catheter - Friend, Foe, or Accomplice?. *JAMA,* 18. Juli 2001, vol. 286 (3), 348-350
- **POLANCZYK CA ; ROHDE LE ; GOLDMAN L ; COOK EF ; THOMAS EJ ; MARCANTONIO ER ; MANGIONE CM ; LEE TH.** Right Heart Cathertization and Cardiac Complications in Patients Undergoing Noncardiac Surgery. *JAMA,* 18. Juli 2001, vol. 286 (3), 309-314
- **FORTIN J ; NESSLER B ; NESSLER W ; SKRA-BAL F.** *Medizinische Elektrode,* 13. Marz 2001, vol. A 392
- **KUBICEK, W.G. ; I.N.KARNEGIS ; R.P. PATTERSON ; D.A. WITSOE ; R.H. MATTSON.** Development and evaluation of an impedance cardiac output system. *Aerospace Medicine,* 1966, vol. 37, 1208-1212
- **KUBICEK, W.G. ; F.J. KOTTE ; M.U. RAMOS ; R.P. PATTERSON ; D.A. WITSOE ; J.W. LA BREE ; W. REMOLE ; T.E. LAYMAN ; H. SCHOENING ; D. SMITH.** The minnesota impedance cardiograph - theory and applications. *Biomed. Eng.,* Februar 1974, vol. 9 (2), 410-416
- **SRAMEK, B.** Noninvasive technique for measurement of cardiac output by means of electrical impedance. *Proceedings of the Vth ICEBI Tokyo,* 1981
- **SRAMEK, B.BO ; D.M. ROSE ; A. MIYAMOTO.** Stroke volume equation with a linear base impedance model and ist accuracy, as compared to thermodilution and magnetic flowmeter techniques in humans and animals. *Proceedings ofthe Vith ICEBI, Zadar, Yugoslavia,* 1983, 38
- **LAMBERTS, R. ; K.R. VISSER ; W.G. ZIJLSTRA.** *Impedance cardiography.,* 1984
- **BERNSTEIN, D.P.** A new stroke volume equation for thoracic electrical bioimpedance: Theory and rational. *Critical Care Medicin,* 1986, vol. 14, 904-909
- **QUAIL, A.W. ; F.M. TRAUNGOTT ; W.L. PORGES.** Thoracic resistivity for stroke volume calculation in impedance cardiography. *J Appl. Physiol.,* 1981
- **SHANKAR, T.M.R. ; J.G. WEBSTER ; S.Y. SHAO.** The contribution of vessel volume change and resistivity change to the electrical impedance pulse. *IEEE Trans Biomed Engl,* 1985, vol. BME32, 192
- **WANG L ; PATTERSON R.** Multiple Source of the Impedance Cardiogram Based on 3-D Finite Difference Human Thorax Models. *IEEE Transactions on Biomedical Engineering,* 02. Februar 1995, vol. 42 (2), 141-148